# EUROPEAN PATENT APPLICATION

(11) **EP 4 583 020 A1**
(43) Date of publication of application: **09.07.2025**
(21) Application number: 23860151.2
(22) Date of filing: 23.08.2023
(51) Int. Cl.: G06N 20/00, C12M 1/00, C12M 3/00, G06Q 50/04

(54) **INFORMATION PROCESSING DEVICE, OPERATION METHOD FOR INFORMATION PROCESSING DEVICE, AND OPERATION PROGRAM FOR INFORMATION PROCESSING DEVICE**

(30) Priority: 30.08.2022 JP 2022137262
(71) Applicant: FUJIFILM Corporation, Tokyo 106-8620 (JP)
(72) Inventor: KINOSHITA, Junichi, ashigara-shi, Kanagawa 250-0193 (JP); KIKUCHI, Shinichi, ashigara-shi, Kanagawa 250-0193 (JP)
(74) Representative: Meissner Bolte Partnerschaft mbB
(86) International application number: PCT/JP2023/030284
(87) International publication number: WO 2024/048388

(57) **Abstract**

An information processing apparatus includes a processor, in which the processor uses a machine learning model that outputs a prediction value of quality of a divided portion obtained by dividing an entire product in response to input of a manufacturing condition of the divided portion, inputs the manufacturing condition of the divided portion to the machine learning model and outputs the prediction value of the divided portion from the machine learning model, and derives a suitable manufacturing condition under which quality of the entire product reaches a target value by solving an optimization problem seeking a manufacturing condition under which a value of an objective function having a term including a difference between the prediction value of the divided portion and a target value of the quality of the divided portion is minimized.

## Description

### BACKGROUND OF THE INVENTION

### 1. Field of the Invention

The technology of the present disclosure relates to an information processing apparatus, an operation method of an information processing apparatus, and an operation program of an information processing apparatus.

### 2. Description of the Related Art

In the related art, in the manufacture of a product, a large number of prototypes are manufactured by variously changing manufacturing conditions, and the quality of the prototypes is evaluated to search for suitable manufacturing conditions through trial and error. For example, in the manufacture of an endoscope flexible tube using an extrusion molding machine as disclosed in JP2021-166723A, an elasticity value and the like of a large number of prototypes in which an extrusion amount per unit time of a resin material of an inner layer and an outer layer, which cover a flexible tube substrate and have a different thickness ratio in an axial direction, is changed are evaluated to search for a suitable extrusion amount per unit time of the resin material of the inner layer and the outer layer.

### SUMMARY OF THE INVENTION

In the method of searching for suitable manufacturing conditions through trial and error in which a large number of prototypes are manufactured by variously changing manufacturing conditions as described above, a great deal of work and cost are required. Therefore, a method of deriving suitable manufacturing conditions under which the quality is a target value by solving an optimization problem using a machine learning model that outputs a prediction value of the quality of the product in response to input of the manufacturing conditions is considered.

However, in a case in which the machine learning model is a model specialized for one type of product, there is a problem with general-purpose properties in that the model cannot be applied to another type of product. Taking an endoscope flexible tube as an example, a machine learning model specialized for a large-diameter long flexible tube of an oral endoscope for an upper digestive tract cannot derive suitable manufacturing conditions of a small-diameter short flexible tube of a transnasal endoscope for bronchi.

One embodiment according to the technology of the present disclosure provides an information processing apparatus, an operation method of an information processing apparatus, and an operation program of an information processing apparatus capable of deriving a general-purpose and suitable manufacturing condition regardless of a type of a product.

An information processing apparatus of the present disclosure comprises: a processor, in which the processor uses a machine learning model that outputs a prediction value of quality of a divided portion obtained by dividing an entire product in response to input of a manufacturing condition of the divided portion, inputs the manufacturing condition of the divided portion to the machine learning model and outputs the prediction value of the divided portion from the machine learning model, and derives a suitable manufacturing condition under which quality of the entire product reaches a target value by solving an optimization problem seeking a manufacturing condition under which a value of an objective function having a term including a difference between the prediction value of the divided portion and a target value of the quality of the divided portion is minimized.

It is preferable that the objective function has a regularization term for accommodating the manufacturing condition of the divided portion in a constraint condition related to a manufacturing condition of the entire product.

It is preferable that the regularization term is a term for smoothing the manufacturing condition of the divided portion.

It is preferable that the regularization term is a sum of second-order differential values of the manufacturing condition of the divided portion.

It is preferable that the term including the difference is a sum of values obtained by dividing the difference by the target value of the divided portion.

It is preferable that at least one of physical property information of a material constituting the product or design information of the product is also input to the machine learning model.

It is preferable that there are a plurality of types of the prediction values, and the machine learning model is prepared for each of the plurality of types of the prediction values.

It is preferable that the product is an endoscope flexible tube including a flexible tube substrate and a resin layer that covers the flexible tube substrate and that is composed of an inner layer and an outer layer of which a thickness ratio is different in an axial direction, the divided portion is a portion obtained by dividing the endoscope flexible tube along the axial direction, the manufacturing condition includes an extrusion amount per unit time of a resin material of the inner layer and an extrusion amount per unit time of a resin material of the outer layer, as obtained from an extrusion molding machine, and the prediction value includes an elasticity value of the divided portion, a thickness of the inner layer of the divided portion, and a thickness of the outer layer of the divided portion.

It is preferable that the product is a sheet obtained by applying a liquid to a long support to be transported, the divided portion is a portion obtained by dividing the sheet along a width direction, the manufacturing condition includes a control amount of a tension adjusting roller that is disposed on an upstream side of an application location of the liquid and that adjusts a tension applied to the support at the application location, and the prediction value includes a coating thickness of the liquid in the divided portion.

It is preferable that the product is a cell cultured in a culture tank, the divided portion is a portion obtained by dividing the culture tank, the manufacturing condition includes a culture condition of the cell, and the prediction value includes an index value indicating desirability of a culture environment for the cell.

An operation method of an information processing apparatus of the present disclosure comprises: using a machine learning model that outputs a prediction value of quality of a divided portion obtained by dividing an entire product in response to input of a manufacturing condition of the divided portion; inputting the manufacturing condition of the divided portion to the machine learning model and outputting the prediction value of the divided portion from the machine learning model; and deriving a suitable manufacturing condition under which quality of the entire product reaches a target value by solving an optimization problem seeking a manufacturing condition under which a value of an objective function having a term including a difference between the prediction value of the divided portion and a target value of the quality of the divided portion is minimized.

An operation program of an information processing apparatus of the present disclosure causes a computer to execute: using a machine learning model that outputs a prediction value of quality of a divided portion obtained by dividing an entire product in response to input of a manufacturing condition of the divided portion; inputting the manufacturing condition of the divided portion to the machine learning model and outputting the prediction value of the divided portion from the machine learning model; and deriving a suitable manufacturing condition under which quality of the entire product reaches a target value by solving an optimization problem seeking a manufacturing condition under which a value of an objective function having a term including a difference between the prediction value of the divided portion and a target value of the quality of the divided portion is minimized.

According to the technology of the present disclosure, it is possible to provide an information processing apparatus, an operation method of an information processing apparatus, and an operation program of an information processing apparatus capable of deriving a general-purpose and suitable manufacturing condition regardless of a type of a product.

### BRIEF DESCRIPTION OF THE DRAWINGS

Fig. 1 is a diagram showing an information processing apparatus, an extrusion molding machine, and an endoscope flexible tube.
Fig. 2 is a diagram showing the extrusion molding machine.
Fig. 3 is a diagram showing a divided portion treated as one unit for predicting quality.
Fig. 4 is a block diagram showing a computer that constitutes the information processing apparatus.
Fig. 5 is a block diagram showing a processing unit of a CPU of the information processing apparatus.
Fig. 6 is a diagram showing input data.
Fig. 7 is a diagram showing a target value group.
Fig. 8 is a graph showing formation of an elasticity standard value.
Fig. 9 is a diagram showing a prediction model group.
Fig. 10 is a diagram showing processing of a prediction unit with respect to an elasticity value prediction model.
Fig. 11 is a diagram showing processing of the prediction unit with respect to an inner layer thickness prediction model.
Fig. 12 is a diagram showing processing of the prediction unit with respect to an outer layer thickness prediction model.
Fig. 13 is a diagram showing processing of the prediction unit that obtains a total thickness prediction value from an inner layer thickness prediction value and an outer layer thickness prediction value.
Fig. 14 is a diagram showing a prediction value group.
Fig. 15 is a diagram showing learning data.
Fig. 16 is a diagram showing processing in a learning phase of the elasticity value prediction model.
Fig. 17 is a diagram showing processing in a learning phase of the inner layer thickness prediction model.
Fig. 18 is a diagram showing processing in a learning phase of the outer layer thickness prediction model.
Fig. 19 is a diagram showing an objective function.
Fig. 20 is a diagram showing processing of a derivation unit and suitable manufacturing conditions.
Fig. 21 is a flowchart showing a processing procedure of the information processing apparatus.
Fig. 22 is a diagram showing a state in which suitable manufacturing conditions of a large-diameter long flexible endoscope flexible tube are derived.
Fig. 23 is a diagram showing a state in which suitable manufacturing conditions of a small-diameter short flexible endoscope flexible tube are derived.
Fig. 24 is a view showing a main part of a sheet manufacturing apparatus according to a second embodiment.
Fig. 25 is a diagram showing a divided portion of the second embodiment.
Fig. 26 is a diagram showing input data of the second embodiment.
Fig. 27 is a diagram showing a prediction model group of the second embodiment.
Fig. 28 is a diagram showing processing of the prediction unit with respect to a tension prediction model.
Fig. 29 is a diagram showing processing of the prediction unit with respect to a hydraulic pressure prediction model.
Fig. 30 is a diagram showing processing of the prediction unit with respect to a coating thickness prediction model.
Fig. 31 is a diagram showing a prediction value group of the second embodiment.
Fig. 32 is a diagram showing an objective function of the second embodiment.
Fig. 33 is a diagram showing processing of the derivation unit and suitable manufacturing conditions of the second embodiment.
Fig. 34 is a diagram showing a culture tank and a cell separation filter.
Fig. 35 is a diagram showing a divided portion of a third embodiment.
Fig. 36 is a diagram showing input data of the third embodiment.
Fig. 37 is a diagram showing processing of the prediction unit with respect to a culture environment prediction model.
Fig. 38 is a diagram showing a prediction value group of the third embodiment.
Fig. 39 is a diagram showing an objective function of the third embodiment.
Fig. 40 is a diagram showing processing of the derivation unit and provisional suitable manufacturing conditions of the third embodiment.
Fig. 41 is a diagram showing a state in which a more suitable oxygen supply amount is derived from an oxygen concentration of the provisional suitable manufacturing conditions.

### DESCRIPTION OF THE PREFERRED EMBODIMENTS

### [First Embodiment]

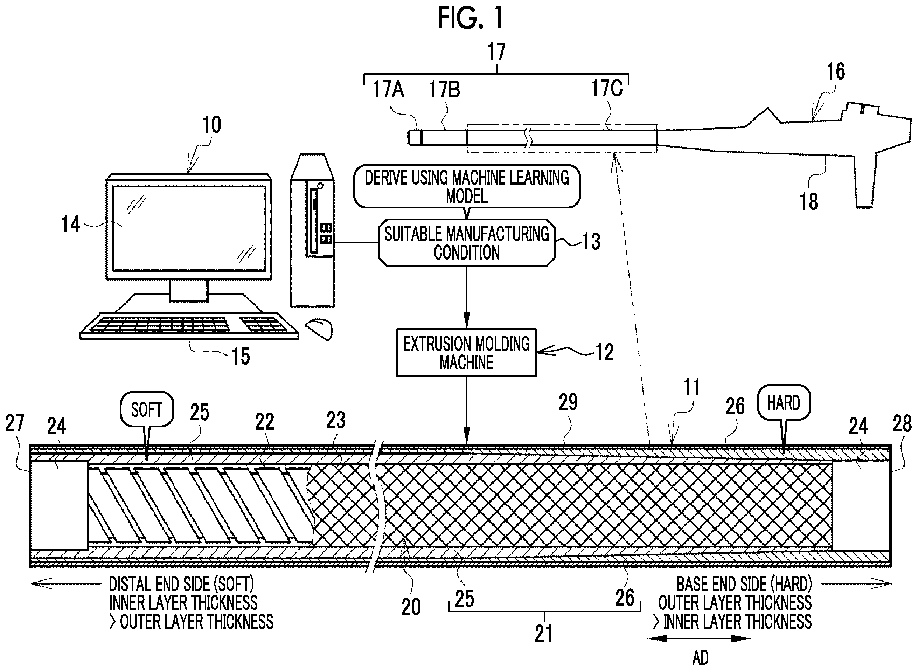

As shown in Fig. 1 as an example, an information processing apparatus 10 outputs a suitable manufacturing condition 13 of an endoscope flexible tube 11 to an extrusion molding machine 12 that manufactures the endoscope flexible tube 11. The suitable manufacturing condition 13 is derived using a machine learning model as described below, and is a manufacturing condition under which the quality of the entire endoscope flexible tube 11 reaches a target value. Here, the phrase "the quality reaches a target value" includes not only a case in which the quality completely matches the target value but also a case in which the quality falls within a range of the target value ± α (α is an allowable error).

The information processing apparatus 10 is, for example, a desktop personal computer, and includes a display 14 that displays various screens and an input device 15 such as a keyboard, a mouse, a touch panel, or a microphone for voice input. The information processing apparatus 10 is operated by, for example, an operator of the extrusion molding machine 12. The information processing apparatus 10 and the extrusion molding machine 12 are communicatively connected to each other via a communication network such as a local area network (LAN).

The endoscope flexible tube 11 constitutes a part of an endoscope 16. More specifically, the endoscope 16 has an insertion part 17 to be inserted into a body cavity. The insertion part 17 has a distal end part 17A, a bendable part 17B, and a soft part 17C. An imaging element for in-body cavity imaging is incorporated in the distal end part 17A. The bendable part 17B is consecutively installed to the distal end part 17A and is bent up, down, left, and right to change an orientation of the distal end part 17A. The soft part 17C is a soft and elongated tubular portion that connects the bendable part 17B and a hand operating part 18 in which an operation knob or the like for operating the bendable part 17B is provided, and occupies most of the insertion part 17. The endoscope flexible tube 11 constitutes the soft part 17C. The endoscope flexible tube 11 is an example of a "product" according to the technology of the present disclosure.

The endoscope flexible tube 11 is formed by coating an outer peripheral surface of a flexible tube substrate 20 with a resin layer 21. The flexible tube substrate 20 has a configuration in which a spiral tube 22 is covered with a cylindrical mesh body 23. The spiral tube 22 is formed by spirally winding a metal strip piece such as stainless steel. The cylindrical mesh body 23 is formed by braiding metal wires such as stainless steel fibers. A mouthpiece 24 is fitted to both ends of the flexible tube substrate 20. An outer diameter of the endoscope flexible tube 11 is, for example, 10 mm to 14 mm, and a length thereof is, for example, 50 cm to 150 cm.

The resin layer 21 is continuously molded on a surface of the flexible tube substrate 20 by the extrusion molding machine 12. The resin layer 21 has a two-layer structure in which an inner layer 25 that covers the entire circumferential surface around an axis of the flexible tube substrate 20 and an outer layer 26 that covers the entire circumferential surface of the inner layer 25 around the axis are laminated. The inner layer 25 is relatively soft, and the outer layer 26 is harder than the inner layer 25. As a resin material of the inner layer 25 and the outer layer 26, for example, a resin containing a polyurethane elastomer as a main component is used.

A thickness of the resin layer 21 formed of the inner layer 25 and the outer layer 26 is substantially the same in an axial direction AD of the flexible tube substrate 20, and is, for example, 0.2 mm to 1.0 mm. Note that a thickness ratio of the inner layer 25 to the outer layer 26 is different in the axial direction AD. Specifically, on a distal end 27 side of the flexible tube substrate 20 that is consecutively installed to the bendable part 17B, the inner layer 25 is thicker than the outer layer 26. On the other hand, on a base end 28 side of the flexible tube substrate 20 that is consecutively installed to the hand operating part 18, the outer layer 26 is thicker than the inner layer 25. In addition, the thickness of the inner layer 25 gradually decreases and the thickness of the outer layer 26 gradually increases from the distal end 27 toward the base end 28. The thickness ratio of the inner layer 25 to the outer layer 26 at the distal end 27 is, for example, inner layer: outer layer = 95:5 to 60:40. Meanwhile, the thickness ratio of the inner layer 25 to the outer layer 26 at the base end 28 is, for example, inner layer:outer layer = 5:95 to 40:60. By making the thickness ratio of the inner layer 25 to the outer layer 26 different in this way, the endoscope flexible tube 11 and thus the soft part 17C are soft on the distal end 27 side and are hard on the base end 28 side. As a result, the insertion part 17 is easily inserted into the body cavity.

An outer surface of the resin layer 21 is further coated with a topcoat layer 29. As a material of the topcoat layer 29, any material that does not affect a human body, has chemical resistance, and can withstand a high temperature of steam sterilization may be used, and for example, a fluorine paint is used. A thickness of the topcoat layer 29 is, for example, 10 µm to 200 µm. Gradations or the like indicating a length of the insertion part 17 are printed on a surface of the topcoat layer 29.

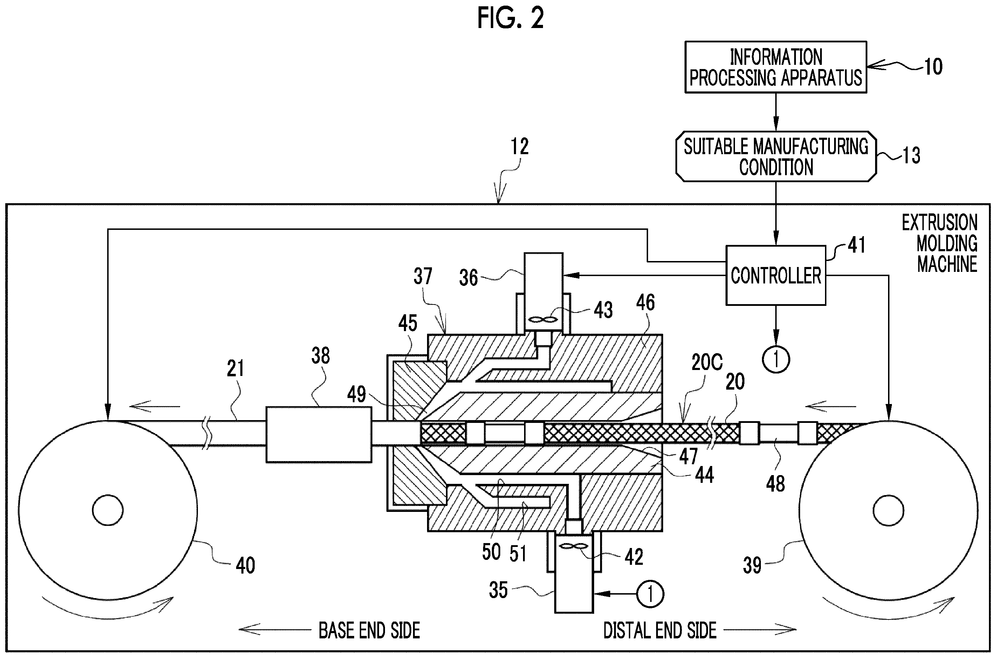

As shown in Fig. 2 as an example, the extrusion molding machine 12 includes extrusion portions 35 and 36, a head portion 37, a cooling portion 38, a delivery drum 39, a winding drum 40, and a controller 41. The extrusion portion 35 includes a hopper (not shown), a screw 42, and the like. The extrusion portion 35 extrudes the resin material of the inner layer 25 in a molten state toward the head portion 37. The extrusion portion 36 includes a hopper (not shown), a screw 43, and the like, similarly to the extrusion portion 35. The extrusion portion 36 extrudes the resin material of the outer layer 26 in a molten state toward the head portion 37. By changing rotation speeds of the screws 42 and 43, an extrusion amount per unit time of the resin material of the inner layer 25 and an extrusion amount per unit time of the resin material of the outer layer 26, and thus the thicknesses of the inner layer 25 and the outer layer 26 can be changed.

The head portion 37 is composed of a nipple 44, a die 45, and a support 46 that fixedly supports the nipple 44 and the die 45. A round hole-like molding passage 47 is formed in a central portion of the nipple 44. An inlet of the molding passage 47 is expanded in a tapered shape. A flexible tube substrate connected body 20C is inserted into the molding passage 47. The flexible tube substrate connected body 20C is obtained by connecting a plurality of flexible tube substrates 20 with connecting members 48. The flexible tube substrate connected body 20C is introduced into the molding passage 47 in a state in which the base end 28 of the flexible tube substrate 20 is in a leading position and the distal end 27 is in a trailing position. A pore diameter of the molding passage 47 is slightly larger than an outer diameter of the flexible tube substrate 20. Conversely, the flexible tube substrate connected body 20C may be introduced into the molding passage 47 in a state in which the distal end 27 of the flexible tube substrate 20 is in the leading position and the base end 28 is in the trailing position.

The inside of the die 45 is heated to a predetermined molding temperature, for example, about 150°C to 300°C by a heater (not shown). A resin passage 49 is formed between the nipple 44 and the die 45 to surround the entire periphery of the molding passage 47. The resin passage 49 communicates with gates 50 and 51 formed in the support 46. The gates 50 and 51 are also formed to surround the entire periphery of the molding passage 47, similarly to the resin passage 49. The resin material of the inner layer 25 and the resin material of the outer layer 26 are extruded to the gates 50 and 51, and are supplied to the molding passage 47 through the resin passage 49 with the resin material of the inner layer 25 on the bottom and the resin material of the outer layer 26 on the top.

The cooling portion 38 is, for example, a water tank in which cooling water is stored. The cooling portion 38 cools the resin material of the inner layer 25 and the resin material of the outer layer 26 that are extrusion-molded in the head portion 37 into the flexible tube substrate connected body 20C.

The flexible tube substrate connected body 20C before extrusion molding is wound around the delivery drum 39. Meanwhile, the flexible tube substrate connected body 20C after extrusion molding is wound around the winding drum 40. A motor (not shown) is connected to the delivery drum 39 and the winding drum 40, and the delivery drum 39 and the winding drum 40 rotate in response to the drive of the motor. As the delivery drum 39 and the winding drum 40 rotate, the flexible tube substrate connected body 20C is delivered from the delivery drum 39 toward the winding drum 40 (the head portion 37 and the cooling portion 38).

A transportation speed of the flexible tube substrate connected body 20C by means of the delivery drum 39 and the winding drum 40 is constant. In this way, in a case in which the resin material of the inner layer 25 and the resin material of the outer layer 26 are constantly extrusion-molded into the flexible tube substrate connected body 20C transported at a constant transportation speed, the resin layer 21 can be continuously molded on the plurality of flexible tube substrates 20.

The suitable manufacturing condition 13 from the information processing apparatus 10 is input to the controller 41. The controller 41 controls the operation of each part of the extrusion molding machine 12 according to the suitable manufacturing condition 13. The controller 41 controls the rotation speeds of the screws 42 and 43, thereby controlling the extrusion amount per unit time of the resin material of the inner layer 25 and the extrusion amount per unit time of the resin material of the outer layer 26. In addition, the controller 41 controls the drive of the motor for the delivery drum 39 and the winding drum 40, thereby maintaining the transportation speed of the flexible tube substrate connected body 20C constant. In addition, the controller 41 controls the drive of the heater for the die 45, thereby controlling the molding temperature or maintaining the temperature of the cooling water of the cooling portion 38 constant.

In a case in which the resin layer 21 is molded on one flexible tube substrate 20, the controller 41 performs control such that the thickness of the outer layer 26 is larger than the thickness of the inner layer 25 at the base end 28, the thickness of the outer layer 26 is gradually decreased and the thickness of the inner layer 25 is gradually increased toward the distal end 27, and the thickness of the inner layer 25 is larger than the thickness of the outer layer 26 at the distal end 27. After completing the extrusion molding on one flexible tube substrate 20, the controller 41 switches the extrusion amount per unit time of the resin material of the inner layer 25 and the extrusion amount per unit time of the resin material of the outer layer 26 in a portion of the connecting member 48 in order to prepare for the extrusion molding on the next flexible tube substrate 20.

The flexible tube substrate connected body 20C wound around the winding drum 40 has the connecting member 48 removed and is separated into the individual flexible tube substrates 20. Then, each flexible tube substrate 20 is coated with the topcoat layer 29. Finally, the mouthpiece 24 is attached to the distal end 27 and the base end 28 of each flexible tube substrate 20. Accordingly, the endoscope flexible tube 11 is completed. The endoscope flexible tube 11 is then transferred to an assembly process of the endoscope 16.

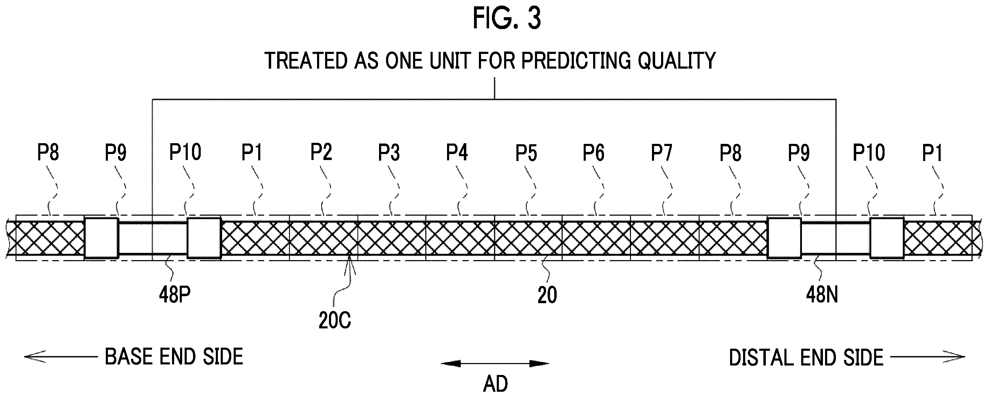

As shown in Fig. 3 as an example, the information processing apparatus 10 treats divided portions P1, P2, P3, P4, P5, P6, P7, and P8 (arranged in this order from the base end 28 side), which are portions obtained by dividing one flexible tube substrate 20 along the axial direction AD, and divided portions P9 and P10 (arranged in this order from the base end 28 side), which are portions obtained by dividing the connecting member 48 along the axial direction, as one unit for predicting the quality. Here, the divided portion P9 treated as one unit for predicting the quality is a portion of a connecting member 48N that connects the flexible tube substrate 20 and the next flexible tube substrate 20. In addition, the divided portion P10 treated as one unit for predicting the quality is a portion of a connecting member 48P that connects the flexible tube substrate 20 and the previous flexible tube substrate 20. Lengths of the divided portions P1 to P10 may be the same as or different from each other. Hereinafter, in a case in which there is no need to particularly distinguish between the divided portions P1 to P10, the divided portions may be referred to as a divided portion P.

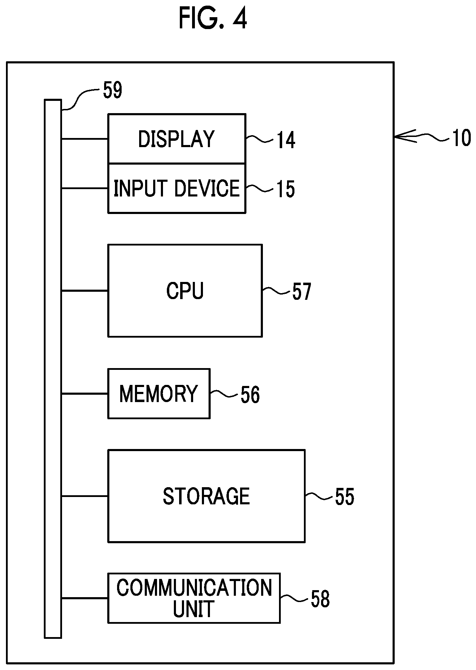

As shown in Fig. 4 as an example, the computer constituting the information processing apparatus 10 comprises a storage 55, a memory 56, a central processing unit (CPU) 57, and a communication unit 58, in addition to the display 14 and the input device 15 described above. These units are connected to each other via a busline 59.

The storage 55 is a hard disk drive that is incorporated in the computer constituting the information processing apparatus 10 or connected to the computer through a cable or a network. Of course, the storage 55 is a disk array in which a plurality of hard disk drives are mounted. In the storage 55, a control program, such as an operating system, various application programs, various data associated with such programs, and the like are stored. A solid state drive may be used instead of the hard disk drive.

The memory 56 is a work memory for the CPU 57 to execute processing. The CPU 57 loads the program stored in the storage 55 into the memory 56 and executes the processing in accordance with the program. As a result, the CPU 57 controls each unit of the computer in an integrated manner. The CPU 57 is an example of a "processor" according to the technology of the present disclosure. The memory 56 may be built in the CPU 57. The communication unit 58 controls transmission of various types of information to an external device such as the extrusion molding machine 12.

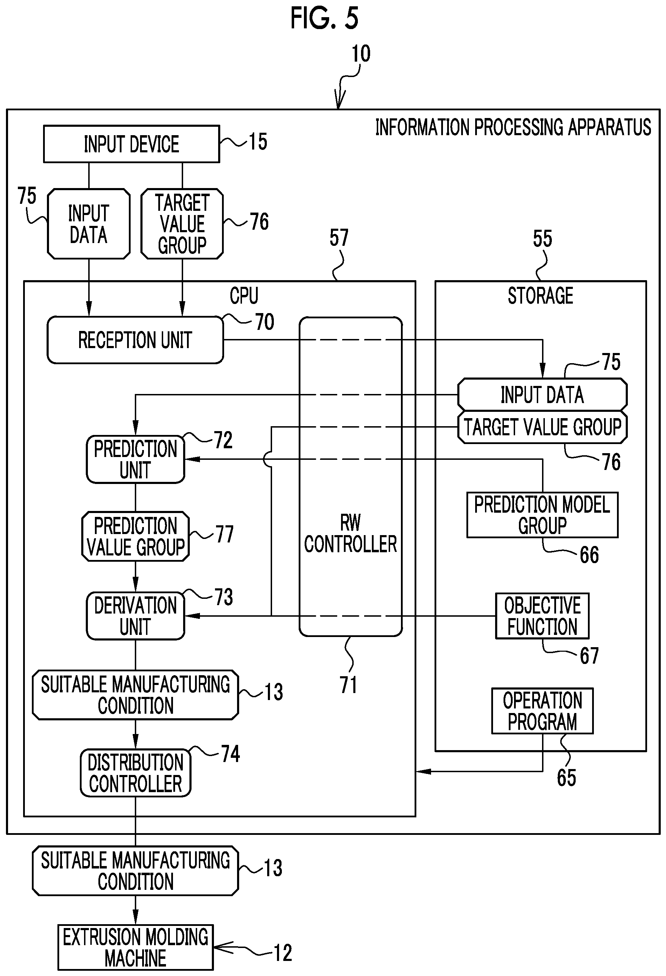

As shown in Fig. 5 as an example, an operation program 65 is stored in the storage 55 of the information processing apparatus 10. The operation program 65 is an application program for causing the computer to function as the information processing apparatus 10. That is, the operation program 65 is an example of an "operation program of an information processing apparatus" according to the technology of the present disclosure. The storage 55 also stores a prediction model group 66, an objective function 67, and the like.

In a case in which the operation program 65 is activated, the CPU 57 of the computer constituting the information processing apparatus 10 functions as a reception unit 70, a read/write (hereinafter, abbreviated as RW) controller 71, a prediction unit 72, a derivation unit 73, and a distribution controller 74 in cooperation with the memory 56 and the like.

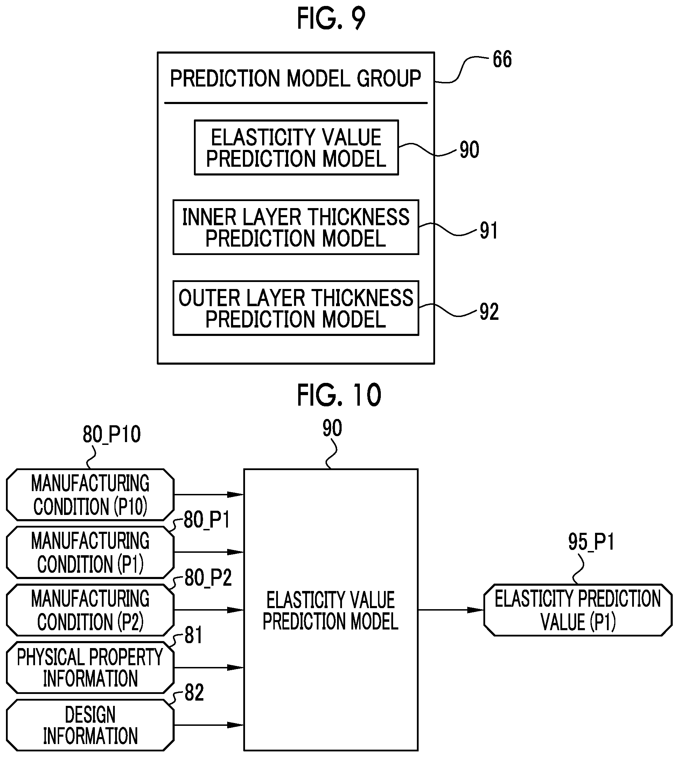

The reception unit 70 receives various types of information input by the operator via the input device 15. For example, the reception unit 70 receives input data 75. The input data 75 is data to be input to an elasticity value prediction model 90, an inner layer thickness prediction model 91, and an outer layer thickness prediction model 92 (see Fig. 9) that constitute the prediction model group 66. The reception unit 70 outputs the input data 75 to the RW controller 71.

In addition, the reception unit 70 receives a target value group 76. The target value group 76 is a set of target values of the quality of each divided portion P. The reception unit 70 outputs the target value group 76 to the RW controller 71. Although not shown, the reception unit 70 also receives a derivation instruction of the suitable manufacturing condition 13, a distribution instruction of the suitable manufacturing condition 13, and the like.

The RW controller 71 controls the storage of various types of information in the storage 55 and the readout of various types of information in the storage 55. For example, the RW controller 71 stores the input data 75 and the target value group 76 from the reception unit 70 in the storage 55. In addition, the RW controller 71 reads out the input data 75 from the storage 55 and outputs the read out input data 75 to the prediction unit 72. The RW controller 71 reads out the prediction model group 66 from the storage 55 and outputs the read out prediction model group 66 to the prediction unit 72. Further, the RW controller 71 reads out the objective function 67 and the target value group 76 from the storage 55 and outputs the read out objective function 67 and target value group 76 to the derivation unit 73.

The prediction unit 72 operates in response to a derivation instruction of the suitable manufacturing condition 13. The prediction unit 72 inputs the input data 75 to the elasticity value prediction model 90, the inner layer thickness prediction model 91, and the outer layer thickness prediction model 92 that constitute the prediction model group 66. Then, the prediction values of the quality of the divided portion P are output from the elasticity value prediction model 90, the inner layer thickness prediction model 91, and the outer layer thickness prediction model 92. The prediction unit 72 outputs a prediction value group 77, which is a set of the prediction values output from the elasticity value prediction model 90, the inner layer thickness prediction model 91, and the outer layer thickness prediction model 92, to the derivation unit 73.

The derivation unit 73 operates in response to a derivation instruction of the suitable manufacturing condition 13, similarly to the prediction unit 72. Here, the objective function 67 includes the target value in the target value group 76 and the prediction value in the prediction value group 77 as variables. The derivation unit 73 derives the suitable manufacturing condition 13 by solving an optimization problem seeking a manufacturing condition under which the value of the objective function 67 is minimized by substituting the target value in the target value group 76 and the prediction value in the prediction value group 77 into the objective function 67. The derivation unit 73 outputs the suitable manufacturing condition 13 to the distribution controller 74. As a method of solving the optimization problem, known black-box optimization methods such as a genetic algorithm, an evolution strategy, and Bayesian optimization can be used.

The distribution controller 74 performs control of distributing the suitable manufacturing condition 13 to the extrusion molding machine 12 designated in the distribution instruction of the suitable manufacturing condition 13.

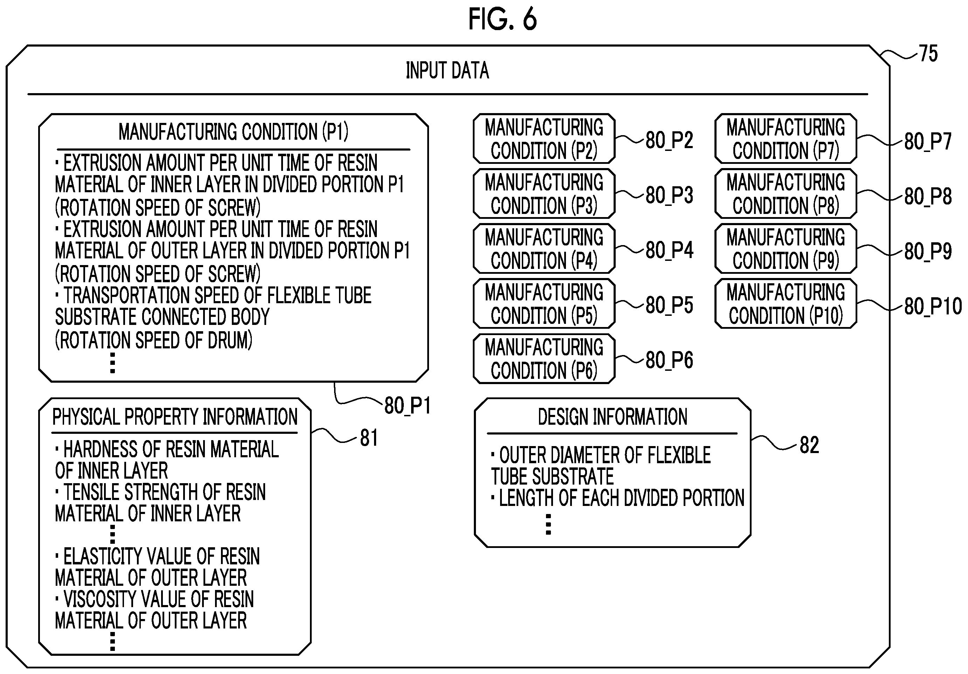

As shown in Fig. 6 as an example, the input data 75 includes a manufacturing condition of each of the divided portions P1 to P10, which are treated as one unit for predicting the quality shown in Fig. 3. More specifically, the input data 75 includes a manufacturing condition 80_P1 of the divided portion P1, a manufacturing condition 80_P2 of the divided portion P2, ..., a manufacturing condition 80_P9 of the divided portion P9, and a manufacturing condition 80_P10 of the divided portion P10. In Figures, the manufacturing condition 80_P1 and the like are denoted by "manufacturing condition (P1)" and the like. The same applies to an elasticity prediction value 95 and the like described below. Hereinafter, in a case in which there is no need to particularly distinguish between the manufacturing conditions 80_P1 to 80_P10 of the divided portions P1 to P10, the manufacturing conditions 80_P1 to 80_P10 may be referred to as a manufacturing condition 80.

The manufacturing condition 80 includes the extrusion amount per unit time of the resin material of the inner layer 25 in the divided portion P (the rotation speed of the screw 42), the extrusion amount per unit time of the resin material of the outer layer 26 in the divided portion P (the rotation speed of the screw 43), the transportation speed of the flexible tube substrate connected body 20C (the rotation speeds of the delivery drum 39 and the winding drum 40), and the like. The extrusion amount per unit time of the resin material of the inner layer 25 and the extrusion amount per unit time of the resin material of the outer layer 26 vary under each manufacturing condition 80 depending on the thicknesses of the inner layer 25 and the outer layer 26 of each divided portion P. On the other hand, the transportation speed of the flexible tube substrate connected body 20C is common to the respective manufacturing conditions 80. The operator inputs the manufacturing condition 80 under which the quality of the entire endoscope flexible tube 11 is considered to be a target value based on the past manufacturing record of the endoscope flexible tube 11 and the operator's own experience. In addition to these, the manufacturing condition 80 may appropriately include an extrusion pressure of each of the resin material of the inner layer 25 and the resin material of the outer layer 26, a molding temperature, and the like. The extrusion pressure and the molding temperature are also common to the respective manufacturing conditions 80, as in the case of the transportation speed of the flexible tube substrate connected body 20C.

The input data 75 includes physical property information 81. The physical property information 81 is information related to physical properties of a material constituting the endoscope flexible tube 11, here, the resin material of the inner layer 25 and the resin material of the outer layer 26. The physical property information 81 includes a hardness (for example, shore A hardness) of the resin material of the inner layer 25, a tensile strength (for example, 100% modulus) of the resin material of the inner layer 25, an elasticity value (for example, bending elastic modulus) of the resin material of the outer layer 26, a viscosity value (for example, melt viscosity) of the resin material of the outer layer 26, and the like. In addition to these, a tensile strength of the resin material of the inner layer 25, an elongation rate of the resin material of the inner layer 25, a viscosity ratio of the resin material of the outer layer 26, and the like may be appropriately added as the physical property information 81.

In addition, the input data 75 includes design information 82. The design information 82 is information related to a design value of the endoscope flexible tube 11. The design information 82 includes the outer diameter of the flexible tube substrate 20, the length of each divided portion P, and the like. The length of each divided portion P may be replaced with a distance between two adjacent divided portions P. In addition to these, amounts of additives to be added to the resin material of the inner layer 25 and the resin material of the outer layer 26 may be appropriately added as the design information 82.

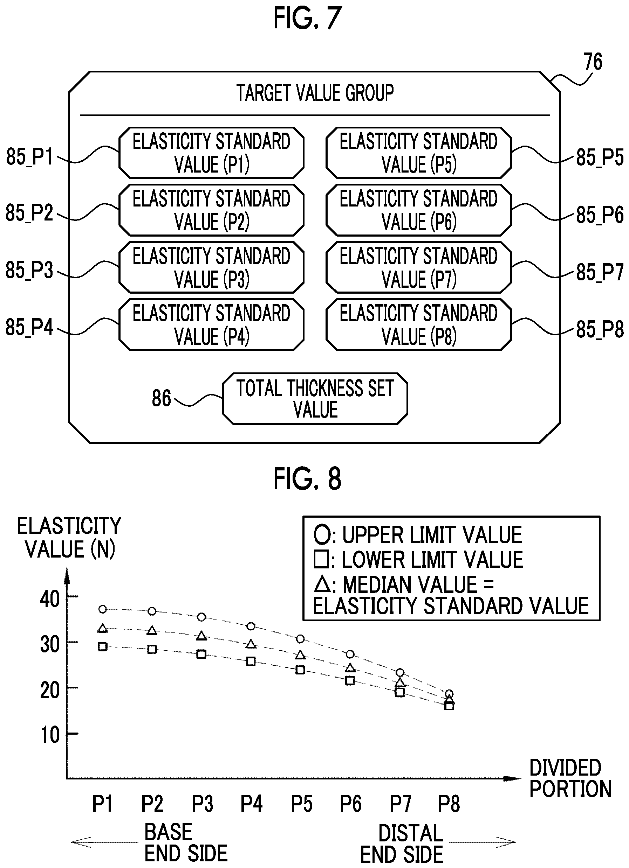

As shown in Fig. 7 as an example, the target value group 76 includes a standard value of the elasticity value of the resin layer 21 of each of the divided portions P1 to P8 (hereinafter, referred to as an elasticity standard value). More specifically, the target value group 76 includes an elasticity standard value 85_P1 of the divided portion P1, an elasticity standard value 85_P2 of the divided portion P2, ..., an elasticity standard value 85_P7 of the divided portion P7, and an elasticity standard value 85_P8 of the divided portion P8. Hereinafter, in a case in which there is no need to particularly distinguish between the elasticity standard values 85_P1 to 85_P8 of the divided portions P1 to P8, the elasticity standard values 85_P1 to 85_P8 may be referred to as an elasticity standard value 85. The elasticity standard value 85 is an example of a "target value" according to the technology of the present disclosure.

In addition, the target value group 76 includes a set value (hereinafter, a total thickness set value) 86 of a total thickness of the resin layer 21 in which the thicknesses of the inner layer 25 and the outer layer 26 are combined. Since the total thickness set value 86 is common to the respective divided portions P, there is only one total thickness set value 86. The total thickness set value 86 is an example of a "target value" according to the technology of the present disclosure, similarly to the elasticity standard value 85.

As shown in Fig. 8 as an example, the elasticity standard value 85 is, for example, a median value between an upper limit value and a lower limit value of the elasticity value of the resin layer 21 of each divided portion P. The upper limit value and the lower limit value of the elasticity value of the resin layer 21 of each divided portion P, and the elasticity standard value 85 of the resin layer 21 of each divided portion P decrease from the divided portion P1 on the base end 28 side to the divided portion P8 on the distal end 27 side. This is due to the fact that the distal end 27 side is relatively soft because the thickness of the inner layer 25 is larger than the thickness of the outer layer 26, and the base end 28 side is relatively hard because the thickness of the outer layer 26 is larger than the thickness of the inner layer 25.

As shown in Fig. 9 as an example, the prediction model group 66 includes the elasticity value prediction model 90, the inner layer thickness prediction model 91, and the outer layer thickness prediction model 92. The elasticity value prediction model 90, the inner layer thickness prediction model 91, and the outer layer thickness prediction model 92 are machine learning models composed of, for example, a neural network. That is, the elasticity value prediction model 90, the inner layer thickness prediction model 91, and the outer layer thickness prediction model 92 are examples of a "machine learning model" according to the technology of the present disclosure. In the following, the elasticity value prediction model 90, the inner layer thickness prediction model 91, and the outer layer thickness prediction model 92 may be collectively referred to as prediction models 90 to 92.

The elasticity value prediction model 90 outputs a prediction value of the elasticity value of the resin layer 21 of the divided portion P (hereinafter, referred to as an elasticity prediction value) in response to the input of the manufacturing condition 80, the physical property information 81, and the design information 82. The inner layer thickness prediction model 91 outputs a prediction value of the thickness of the inner layer 25 of the divided portion P (hereinafter, referred to as an inner layer thickness prediction value) in response to the input of the manufacturing condition 80, the physical property information 81, and the design information 82. The outer layer thickness prediction model 92 outputs a prediction value of the thickness of the outer layer 26 of the divided portion P (hereinafter, referred to as an outer layer thickness prediction value) in response to the input of the manufacturing condition 80, the physical property information 81, and the design information 82. As described above, there are a plurality of types of the prediction values, and the prediction models 90 to 92 are prepared for the plurality of types of the prediction values, respectively.

As shown in Fig. 10 as an example, in a case of predicting the quality of the divided portion P1, the prediction unit 72 inputs the manufacturing condition 80_P1 of the divided portion P1 and the manufacturing conditions 80_P10 and 80_P2 of the divided portion P10 and the divided portion P2 adjacent to both sides of the divided portion P1 to the elasticity value prediction model 90. In addition, the prediction unit 72 inputs the physical property information 81 and the design information 82 to the elasticity value prediction model 90. Then, the prediction unit 72 outputs the elasticity prediction value 95_P1 of the divided portion P1 from the elasticity value prediction model 90. The reason why the manufacturing conditions 80_P10 and 80_P2 are input to the elasticity value prediction model 90 in addition to the manufacturing condition 80_P1 is that it is considered that the elasticity values of the resin layers 21 of the divided portions P10 and P2 adjacent to both sides of the divided portion P1 affect the elasticity value of the resin layer 21 of the divided portion P1.

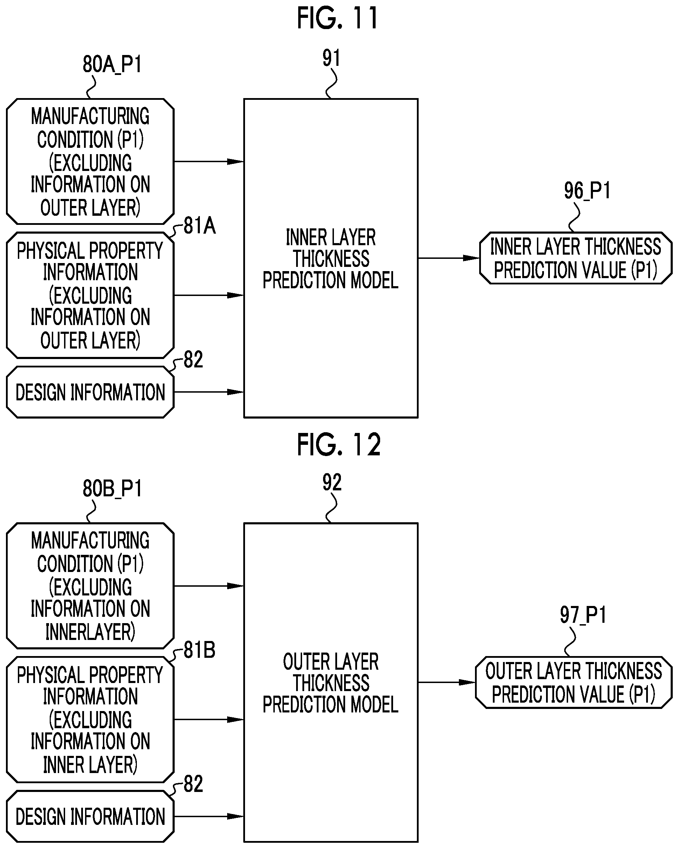

As shown in Fig. 11 as an example, in a case of predicting the quality of the divided portion P1, the prediction unit 72 inputs a manufacturing condition 80A_P1 of the divided portion P1 excluding the information on the outer layer 26, physical property information 81A excluding the information on the outer layer 26, and the design information 82 to the inner layer thickness prediction model 91. Then, the prediction unit 72 outputs an inner layer thickness prediction value 96_P1 of the divided portion P1 from the inner layer thickness prediction model 91. The manufacturing condition 80A_P1 of the divided portion P1 excluding the information on the outer layer 26 is information excluding the extrusion amount per unit time of the resin material of the outer layer 26 in the divided portion P1. In addition, the physical property information 81A excluding the information on the outer layer 26 is information excluding the elasticity value of the resin material of the outer layer 26, the viscosity value of the resin material of the outer layer 26, and the like. The reason why the information on the outer layer 26 is excluded in this way is that the information on the outer layer 26 is considered to be unnecessary for the prediction of the inner layer thickness prediction value.

As shown in Fig. 12 as an example, in a case of predicting the quality of the divided portion P1, the prediction unit 72 inputs a manufacturing condition 80B_P1 of the divided portion P1 excluding the information on the inner layer 25, physical property information 81B excluding the information on the inner layer 25, and the design information 82 to the outer layer thickness prediction model 92. Then, the prediction unit 72 outputs an outer layer thickness prediction value 97_P1 of the divided portion P1 from the outer layer thickness prediction model 92. The manufacturing condition 80B_P1 of the divided portion P1 excluding the information on the inner layer 25 is information excluding the extrusion amount per unit time of the resin material of the inner layer 25 in the divided portion P1. In addition, the physical property information 81B excluding the information on the inner layer 25 is information excluding the hardness of the resin material of the inner layer 25, the tensile strength of the resin material of the inner layer 25, and the like. The reason why the information on the inner layer 25 is excluded in this way is that, as in the case of the inner layer thickness prediction model 91, the information on the inner layer 25 is considered to be unnecessary for the prediction of the outer layer thickness prediction value.

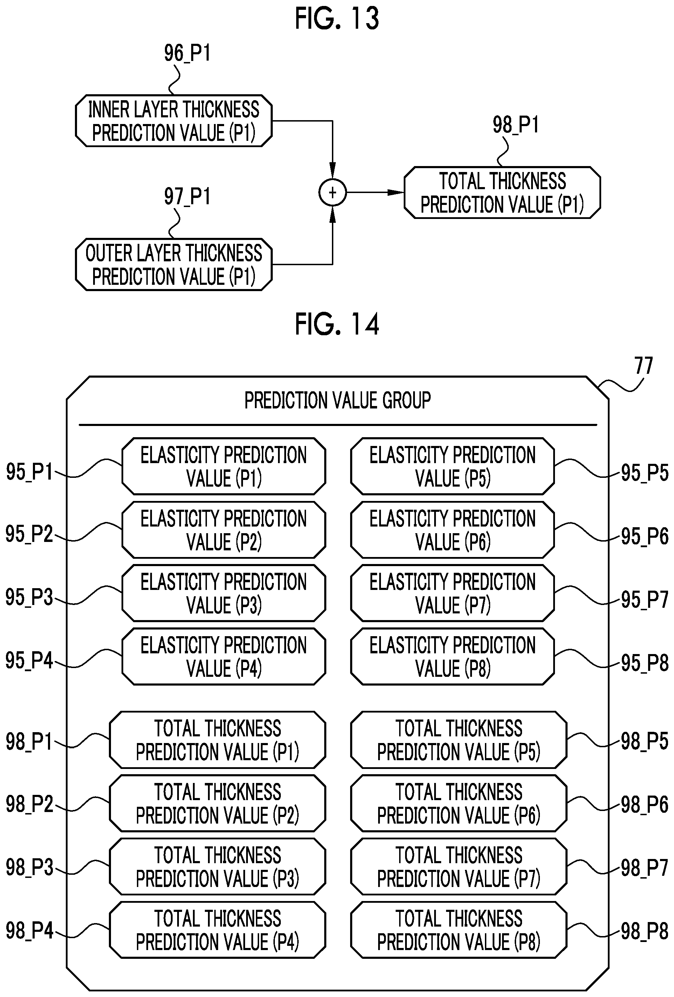

As shown in Fig. 13 as an example, the prediction unit 72 adds up the inner layer thickness prediction value 96_P1 and the outer layer thickness prediction value 97_P1 of the divided portion P1 to obtain a prediction value of a total thickness (hereinafter, referred to as a total thickness prediction value) 98_P1 of the resin layer 21 of the divided portion P1.

In Figs. 10 to 13, an example has been described in which the elasticity prediction value 95_P1, the inner layer thickness prediction value 96_P1, and the outer layer thickness prediction value 97_P1 of the divided portion P1 are output from the prediction models 90 to 92, respectively, to obtain the total thickness prediction value 98_P1 of the divided portion P1, but, similarly for the remaining divided portions P2 to P8, the prediction unit 72 outputs elasticity prediction values 95_P2 to 95_P8 (see Fig. 14), inner layer thickness prediction values 96_P2 to 96_P8 (not shown), and outer layer thickness prediction values 97_P2 to 97_P8 (not shown) from the prediction models 90 to 92, respectively, to obtain total thickness prediction values 98_P2 to 98_P8 (see Fig. 14) of the remaining divided portions P2 to P8. Hereinafter, in a case in which there is no need to particularly distinguish between the elasticity prediction values 95_P1 to 95_P8, the inner layer thickness prediction values 96_P1 to 96_P8, the outer layer thickness prediction values 97_P1 to 97_P8, and the total thickness prediction values 98_P1 to 98_P8, the elasticity prediction values 95_P1 to 95_P8, the inner layer thickness prediction values 96_P1 to 96_P8, the outer layer thickness prediction values 97_P1 to 97_P8, and the total thickness prediction values 98_P1 to 98_P8 may be referred as an elasticity prediction value 95, an inner layer thickness prediction value 96, an outer layer thickness prediction value 97, and a total thickness prediction value 98, respectively. The elasticity prediction value 95, the inner layer thickness prediction value 96, the outer layer thickness prediction value 97, and the total thickness prediction value 98 are examples of a "prediction value" according to the technology of the present disclosure.

By obtaining the elasticity prediction value 95 and the total thickness prediction value 98 of each divided portion P in this way, the prediction value group 77 has a content as shown in Fig. 14 as an example. That is, the prediction value group 77 includes the elasticity prediction value 95_P1 of the divided portion P1, the elasticity prediction value 95_P2 of the divided portion P2, ..., the elasticity prediction value 95_P7 of the divided portion P7, and the elasticity prediction value 95_P8 of the divided portion P8. In addition, the prediction value group 77 includes the total thickness prediction value 98_P1 of the divided portion P1, the total thickness prediction value 98_P2 of the divided portion P2, ..., the total thickness prediction value 98_P7 of the divided portion P7, and the total thickness prediction value 98_P8 of the divided portion P8.

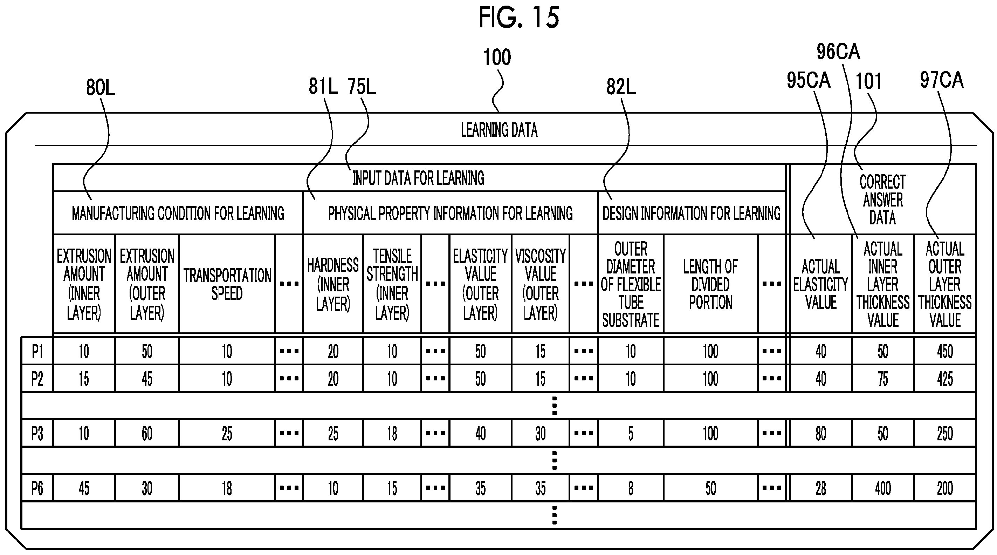

As shown in Fig. 15 as an example, learning data 100 is data collected from the endoscope flexible tube 11 manufactured in the past in order to be used in learning of each of the prediction models 90 to 92. The learning data 100 includes input data for learning 75L and correct answer data 101. The input data for learning 75L is data corresponding to the input data 75. The input data for learning 75L has a manufacturing condition for learning 80L, physical property information for learning 81L, and design information for learning 82L. The manufacturing condition for learning 80L is data corresponding to the manufacturing condition 80, the physical property information for learning 81L is data corresponding to the physical property information 81, and the design information for learning 82L is data corresponding to the design information 82.

As can be seen from the manufacturing condition for learning 80L, the learning data 100 is a set of data related to the divided portion P manufactured at the extrusion amount per unit time of the resin material of the inner layer 25, the extrusion amount per unit time of the resin material of the outer layer 26, and the transportation speed of the flexible tube substrate connected body 20C. In addition, as can be seen from the physical property information for learning 81L, the learning data 100 is also a set of data related to the divided portion P using the inner layer 25 and the outer layer 26 having various physical properties. Further, as can be seen from the design information for learning 82L, the learning data 100 is also a set of data related to the divided portion P of various design values, such as the outer diameter of the flexible tube substrate 20 of 10 mm, 5 mm, and 8 mm, and the length of the divided portion P of 100 mm and 50 mm.

One set of the correct answer data 101 is registered corresponding to each input data for learning 75L. The correct answer data 101 is data for matching an answer, so to speak, with the outputs from the prediction models 90 to 92, that is, the elasticity prediction value 95, the inner layer thickness prediction value 96, and the outer layer thickness prediction value 97. The correct answer data 101 includes an actual measured value (hereinafter, referred to as an actual elasticity value) 95CA of an elasticity value of each divided portion P of the endoscope flexible tube 11 manufactured in the past, an actual measured value (hereinafter, referred to as an actual inner layer thickness value) 96CA of a thickness of the inner layer 25 of each divided portion P of the endoscope flexible tube 11 manufactured in the past, and a measured value (hereinafter, referred to as an actual outer layer thickness value) 97CA of a thickness of the outer layer 26 of each divided portion P of the endoscope flexible tube 11 manufactured in the past.

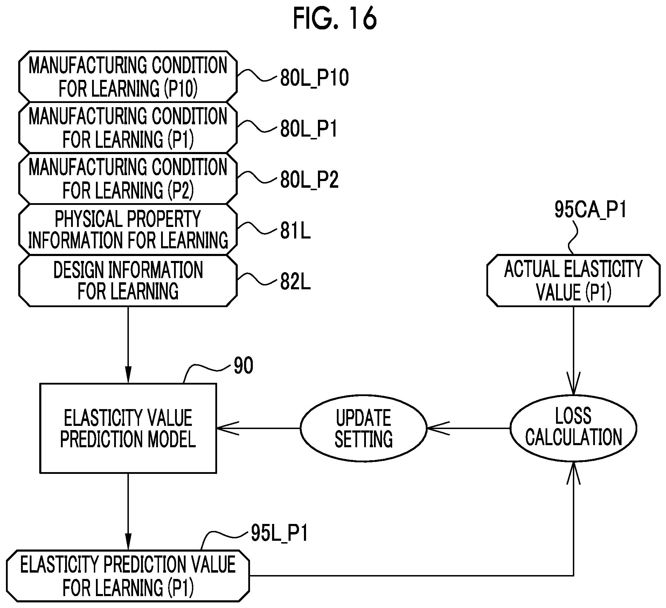

As shown in Figs. 16 to 18 as an example, in a learning phase of each of the prediction models 90 to 92, the input data for learning 75L is given and learning is performed. Specifically, as shown in Fig. 16, in the learning phase of the elasticity value prediction model 90, the manufacturing condition for learning 80L, the physical property information for learning 81L, and the design information for learning 82L are input to the elasticity value prediction model 90. The manufacturing condition for learning 80L is a manufacturing condition for learning 80L of the divided portion P for predicting the elasticity prediction value 95, here, a manufacturing condition for learning 80L_P1 of the divided portion P1. In addition, the manufacturing condition for learning 80L is manufacturing conditions for learning 80L of the divided portions P adjacent to both sides of the divided portion P for predicting the elasticity prediction value 95, here, a manufacturing condition for learning 80L_P10 of the divided portion P10 and a manufacturing condition for learning 80L_P2 of the divided portion P2. In response to the input of such input data for learning 75L, the elasticity value prediction model 90 outputs an elasticity prediction value for learning 95L, here, an elasticity prediction value for learning 95L_P1 of the divided portion P1.

In the learning phase of the elasticity value prediction model 90, a loss calculation of the elasticity value prediction model 90 using a loss function is performed based on the elasticity prediction value for learning 95L, here, the elasticity prediction value for learning 95L_P1 of the divided portion P1, and the actual elasticity value 95CA corresponding to the input data for learning 75L, here, an actual elasticity value 95CA_P1 of the divided portion P1. Then, update of various coefficients (for example, coefficients of convolutional layer filters) of the elasticity value prediction model 90 is set according to a result of the loss calculation, and the elasticity value prediction model 90 is updated according to the update setting.

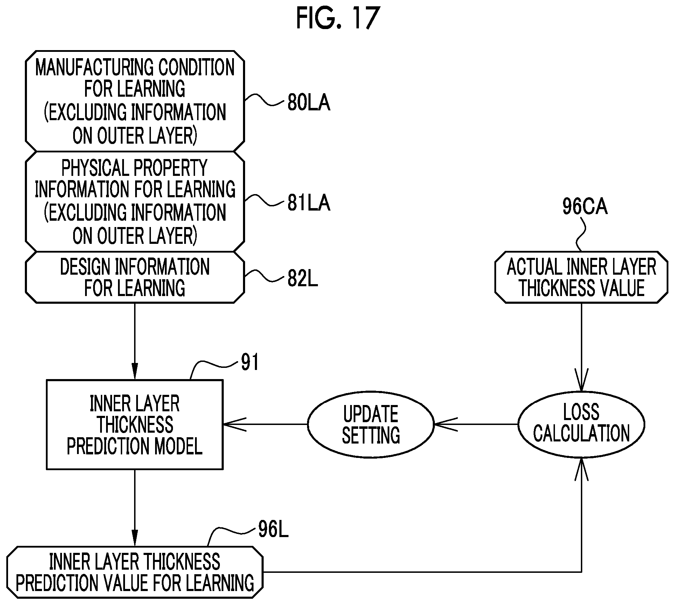

As shown in Fig. 17, in the learning phase of the inner layer thickness prediction model 91, a manufacturing condition for learning 80LA excluding the information on the outer layer 26, physical property information for learning 81LA excluding the information on the outer layer 26, and the design information for learning 82L are input to the inner layer thickness prediction model 91. The inner layer thickness prediction model 91 outputs an inner layer thickness prediction value for learning 96L in response to the input of such input data for learning 75L.

**In** the learning phase of the inner layer thickness prediction model 91, a loss calculation of the inner layer thickness prediction model 91 using a loss function is performed based on the inner layer thickness prediction value for learning 96L and the actual inner layer thickness value 96CA corresponding to the input data for learning 75L. Then, update of various coefficients of the inner layer thickness prediction model 91 is set according to a result of the loss calculation, and the inner layer thickness prediction model 91 is updated according to the update setting.

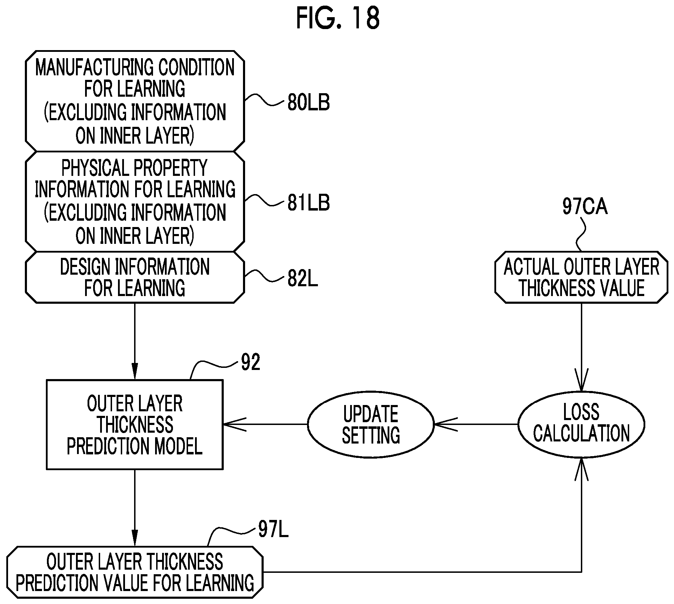

As shown in Fig. 18, in the learning phase of the outer layer thickness prediction model 92, a manufacturing condition for learning 80LB excluding the information on the inner layer 25, physical property information for learning 81LB excluding the information on the inner layer 25, and the design information for learning 82L are input to the outer layer thickness prediction model 92. The outer layer thickness prediction model 92 outputs an outer layer thickness prediction value for learning 97L in response to the input of such input data for learning 75L.

In the learning phase of the outer layer thickness prediction model 92, a loss calculation of the outer layer thickness prediction model 92 using a loss function is performed based on the outer layer thickness prediction value for learning 97L and the actual outer layer thickness value 97CA corresponding to the input data for learning 75L. Then, update of various coefficients of the outer layer thickness prediction model 92 is set according to a result of the loss calculation, and the outer layer thickness prediction model 92 is updated according to the update setting.

In the learning phase of each of the prediction models 90 to 92, the above-mentioned series of processing including the input of the input data for learning 75L, the output of the elasticity prediction value for learning 95L, the inner layer thickness prediction value for learning 96L, and the outer layer thickness prediction value for learning 97L, the loss calculation, the update setting, and the update is repeatedly performed while the input data for learning 75L and the correct answer data 101 are exchanged. The repetition of the series of processing is ended in a case in which the prediction accuracy of the elasticity prediction value for learning 95L, the inner layer thickness prediction value for learning 96L, and the outer layer thickness prediction value for learning 97L reaches a predetermined setting level. Each of the prediction models 90 to 92 of which the prediction accuracy has reached the set level in this way is stored in the storage 55 of the information processing apparatus 10. Regardless of the prediction accuracy of the elasticity prediction value for learning 95L, the inner layer thickness prediction value for learning 96L, and the outer layer thickness prediction value for learning 97L, the learning may be ended in a case in which the series of processing is repeated a set number of times. In addition, the learning may be continuously performed even after the prediction model is stored in the storage 55.

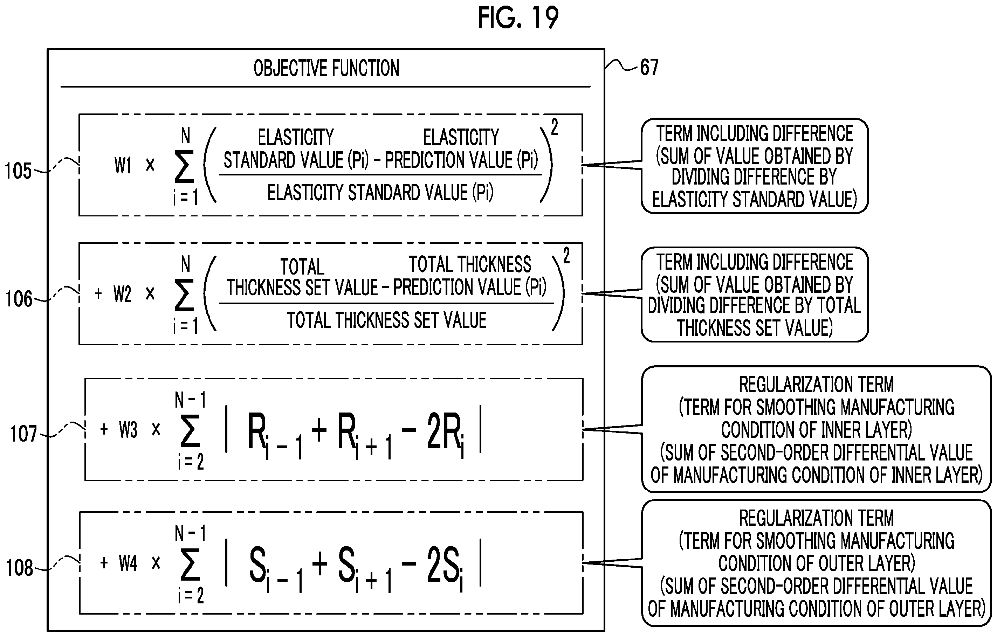

As shown in Fig. 19 as an example, the objective function 67 has a first term 105, a second term 106, a third term 107, and a fourth term 108 that are added together. The first term 105 includes a difference between the elasticity prediction value 95 of each of the divided portions P and the elasticity standard value 85 of each of the divided portions P. More specifically, the first term 105 includes a sum of squares of a value obtained by dividing the difference between the elasticity prediction value 95 and the elasticity standard value 85 by the elasticity standard value 85. The first term 105 is a term obtained by multiplying the sum by a first weight coefficient W1. The first term 105 is an example of a "term including a difference" according to the technology of the present disclosure.

The second term 106 includes a difference between the total thickness prediction value 98 and the total thickness set value 86 of each of the divided portions P. More specifically, the second term 106 includes a sum of squares of a value obtained by dividing the difference between the total thickness prediction value 98 and the total thickness set value 86 by the total thickness set value 86. The second term 106 is a term obtained by multiplying the sum by a second weight coefficient W2. Similarly to the first term 105, the second term 106 is also an example of a "term including a difference" according to the technology of the present disclosure.

The third term 107 and the fourth term 108 are regularization terms for accommodating the manufacturing condition of each of the divided portions P in a constraint condition related to the manufacturing condition of the entire endoscope flexible tube 11. The constraint condition related to the manufacturing condition of the entire endoscope flexible tube 11 is to smoothly connect the manufacturing conditions of the divided portions P in order to smoothly change the elasticity value, the inner layer thickness, and the outer layer thickness throughout the entire endoscope flexible tube 11. Therefore, the third term 107 is a term for smoothing an extrusion amount R per unit time of the resin material of the inner layer 25 of each of the divided portions P. In addition, the fourth term 108 is a term for smoothing an extrusion amount S per unit time of the resin material of the outer layer 26 of each of the divided portions P.

More specifically, the third term 107 includes a sum of second-order differential values of the extrusion amounts R per unit time of the resin material of the inner layer 25 of each of the divided portions P. The third term 107 is a term obtained by multiplying the sum by a third weight coefficient W3. In addition, the fourth term 108 includes a sum of second-order differential values of the extrusion amount S per unit time of the resin material of the outer layer 26 of each of the divided portions P. The fourth term 108 is a term obtained by multiplying the sum by a fourth weight coefficient W4. Here, |Rᵢ₋₁ + Rᵢ₊₁ - 2Rᵢ| of the third term 107 is an approximate expression of the second-order differential of the extrusion amount R per unit time of the resin material of the inner layer 25 of each of the divided portions P. In addition, |Sᵢ₋₁ + Sᵢ₊₁ - 2Sᵢ| of the fourth term 108 is an approximate expression of the second-order differential of the extrusion amount S per unit time of the resin material of the outer layer 26 of each of the divided portions P.

N in the first to fourth terms 105 to 108 is the number of the divided portions P. In addition, the first weight coefficient W1 to the fourth weight coefficient W4 are values (W1 + W2 + W3 + W4 = 1) of which the total is 1. For example, W1 to W4 are 0.25. Note that the first weight coefficient W1 to the fourth weight coefficient W4 do not necessarily have the same value. For example, in a case in which it is desired to place importance on the third term 107, that is, in a case in which it is desired to further smooth the extrusion amount R per unit time of the resin material of the inner layer 25 of each of the divided portions P, the third weight coefficient W3 may be set to a value higher than the others.

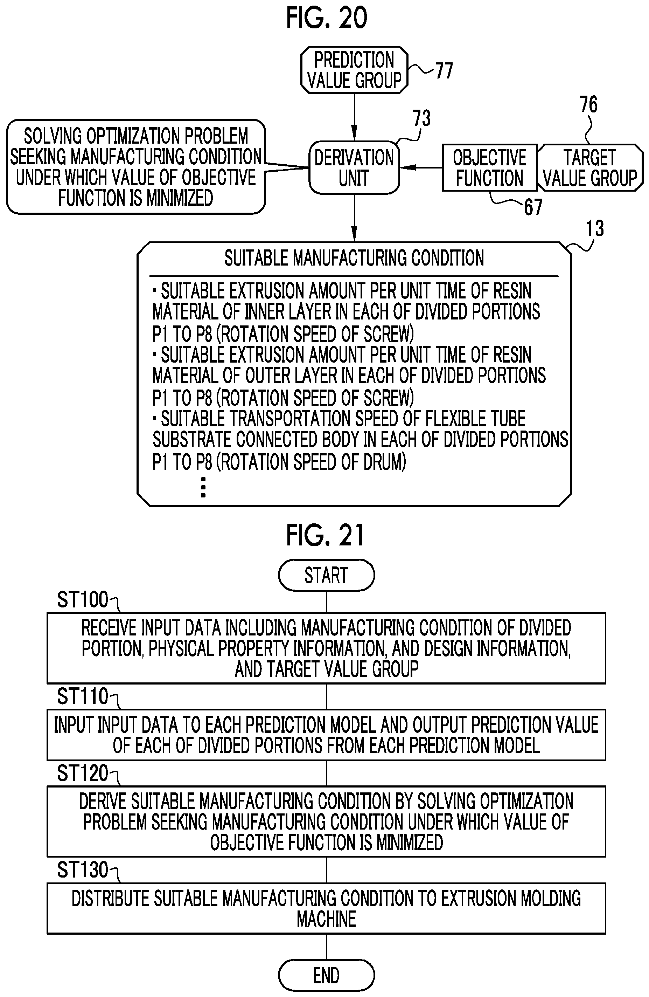

As shown in Fig. 20 as an example, the suitable manufacturing condition 13 derived by the derivation unit 73 by solving an optimization problem seeking a manufacturing condition under which the value of the objective function 67 is minimized includes a suitable extrusion amount per unit time of the resin material of the inner layer 25 in each of the divided portions P1 to P8, a suitable extrusion amount per unit time of the resin material of the outer layer 26 in each of the divided portions P1 to P8, a suitable transportation speed of the flexible tube substrate connected body 20C in each of the divided portions P1 to P8, and the like.

Here, solving the optimization problem seeking the manufacturing condition under which the value of the objective function 67 is minimized means searching for the suitable manufacturing condition 13 under which the difference between the elasticity prediction value 95 and the elasticity standard value 85 of the first term 105 and the difference between the total thickness prediction value 98 and the total thickness set value 86 of the second term 106 are minimized. In addition, solving the optimization problem seeking the manufacturing condition under which the value of the objective function 67 is minimized means searching for the suitable manufacturing condition 13 under which the extrusion amount R per unit time of the resin material of the inner layer 25 of each of the divided portions P and the extrusion amount per unit time of the resin material of the outer layer 26 of each of the divided portions P are smoothed.

Next, an action of the above-described configuration will be described with reference to a flowchart of Fig. 21. First, in a case in which the operation program 65 is activated in the information processing apparatus 10, as shown in Fig. 5, the CPU 57 of the information processing apparatus 10 functions as the reception unit 70, the RW controller 71, the prediction unit 72, the derivation unit 73, and the distribution controller 74.

First, an input screen (not shown) of the input data 75 and the target value group 76 is displayed on the display 14 of the information processing apparatus 10. The operator inputs desired input data 75 and a desired target value group 76 on the input screen. As a result, the input data 75 and the target value group 76 are received by the reception unit 70 (step ST100). The input data 75 and the target value group 76 are output from the reception unit 70 to the RW controller 71. Then, the input data 75 and the target value group 76 are stored in the storage 55 by the RW controller 71. As shown in Fig. 6, the input data 75 includes the manufacturing condition 80, the physical property information 81, and the design information 82 of each of the divided portions P. In addition, as shown in Fig. 7, the target value group 76 includes the elasticity standard value 85 and the total thickness set value 86 of each of the divided portions P. The input data 75 and the target value group 76 may be displayed on different input screens, and the input data 75 and the target value group 76 may be input at different timings.

In a case in which the reception unit 70 receives the derivation instruction of the suitable manufacturing condition 13, the input data 75 is read out from the storage 55 by the RW controller 71 and output to the prediction unit 72. In addition, the target value group 76 is read out from the storage 55 by the RW controller 71 and output to the derivation unit 73.

As shown in Fig. 10, in the prediction unit 72, the input data 75 is input to the elasticity value prediction model 90, and the elasticity prediction value 95 of each of the divided portions P is output from the elasticity value prediction model 90. In addition, as shown in Fig. 11, in the prediction unit 72, the input data 75 is input to the inner layer thickness prediction model 91, and the inner layer thickness prediction value 96 of each of the divided portions P is output from the inner layer thickness prediction model 91. Further, as shown in Fig. 12, in the prediction unit 72, the input data 75 is input to the outer layer thickness prediction model 92, and the outer layer thickness prediction value 97 of each of the divided portions P is output from the outer layer thickness prediction model 92 (step ST110). Then, as shown in Fig. 13, in the prediction unit 72, the inner layer thickness prediction value 96 and the outer layer thickness prediction value 97 are added together, and the total thickness prediction value 98 of each of the divided portions P is obtained. The elasticity prediction value 95 and the total thickness prediction value 98 obtained in this way are output from the prediction unit 72 to the derivation unit 73 as the prediction value group 77 as shown in Fig. 14.

Next, as shown in Fig. 20, the optimization problem seeking the manufacturing condition under which the value of the objective function 67 shown in Fig. 19 is minimized is solved in the derivation unit 73, thereby deriving the suitable manufacturing condition 13 (step ST120). The suitable manufacturing condition 13 is output from the derivation unit 73 to the distribution controller 74.

In a case in which the reception unit 70 receives the distribution instruction of the suitable manufacturing condition 13, the suitable manufacturing condition 13 is distributed to the extrusion molding machine 12 designated by the distribution instruction by the distribution controller 74 (step ST130).

In the extrusion molding machine 12, the suitable manufacturing condition 13 from the information processing apparatus 10 is input to the controller 41. Then, under the control of the controller 41, the operation of each part of the extrusion molding machine 12 is controlled under the suitable manufacturing condition 13, and the endoscope flexible tube 11 is manufactured.

As described above, the CPU 57 of the information processing apparatus 10 functions as the prediction unit 72 and the derivation unit 73. The prediction unit 72 uses the elasticity value prediction model 90, the inner layer thickness prediction model 91, and the outer layer thickness prediction model 92 that output the elasticity prediction value 95, the inner layer thickness prediction value 96, and the outer layer thickness prediction value 97 of the divided portion P in response to the input of the manufacturing condition 80 of the divided portion P obtained by dividing the entire endoscope flexible tube 11. The prediction unit 72 inputs the manufacturing condition 80 of each of the divided portions P to the elasticity value prediction model 90, the inner layer thickness prediction model 91, and the outer layer thickness prediction model 92, and outputs the elasticity prediction value 95, the inner layer thickness prediction value 96, and the outer layer thickness prediction value 97 of each of the divided portions P from the elasticity value prediction model 90, the inner layer thickness prediction model 91, and the outer layer thickness prediction model 92. The derivation unit 73 derives the suitable manufacturing condition 13 under which the quality of the entire endoscope flexible tube 11 reaches the target value by solving the optimization problem seeking the manufacturing condition under which the value of the objective function 67 having the first term 105 including the difference between the elasticity prediction value 95 of each of the divided portions P and the elasticity standard value 85 of each of the divided portions P and the second term 106 including the difference between the total thickness prediction value 98 of each of the divided portions P and the total thickness set value 86 of each of the divided portions P is minimized.

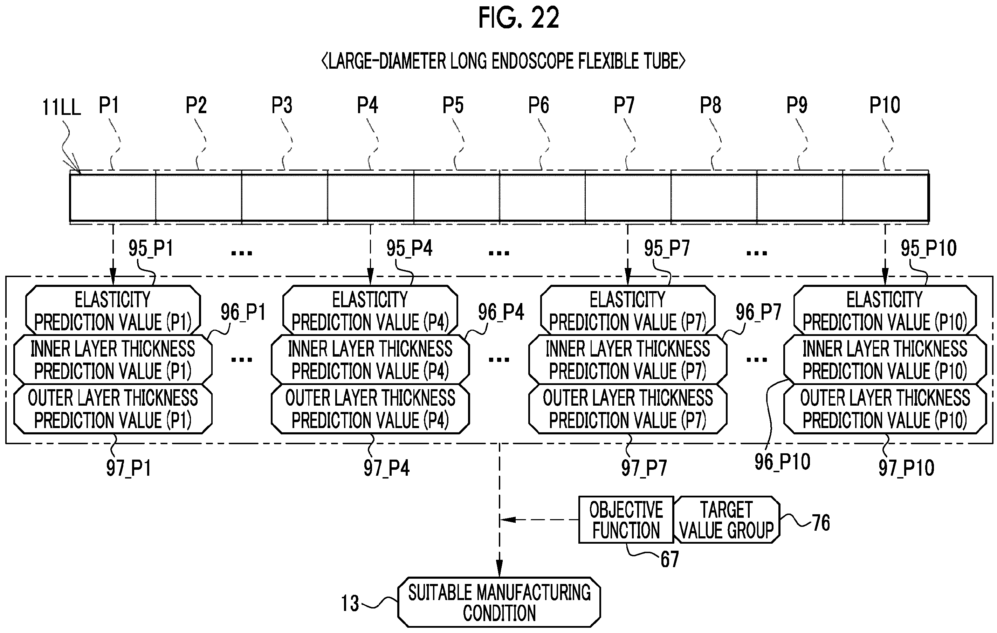

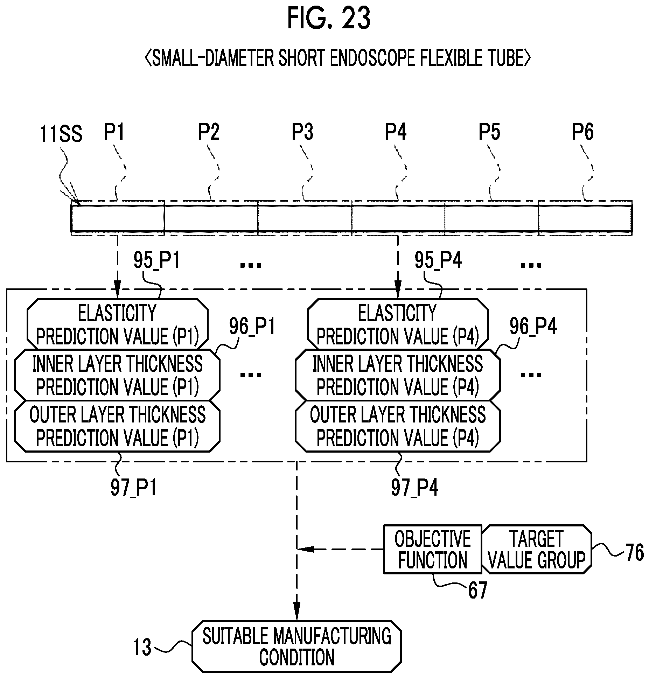

Therefore, as shown in Fig. 22 as an example, for example, for a large-diameter long endoscope flexible tube 11LL of an oral endoscope for an upper digestive tract, the elasticity prediction values 95_P1 to 95_P10 of the divided portions P1 to P10 and the like are output from the elasticity value prediction model 90 and the like to derive a suitable manufacturing condition 13. In addition, as shown in Fig. 23 as an example, for example, for a small-diameter short endoscope flexible tube 11SS of a transnasal endoscope for bronchi, the elasticity prediction values 95_P1 to 95_P6 of the divided portions P1 to P6 and the like are output from the elasticity value prediction model 90 and the like, to derive a suitable manufacturing condition 13.

In a case of predicting the quality of the entire large-diameter long endoscope flexible tube 11LL using a prediction model dedicated to the large-diameter long endoscope flexible tube 11LL, it is necessary to prepare the manufacturing condition of each of the divided portions P1 to P10 as input data. Therefore, the prediction model dedicated to the large-diameter long endoscope flexible tube 11LL cannot predict the quality of the entire small-diameter short endoscope flexible tube 11SS, which has only six divided portions P1 to P6 and lacks the number of the manufacturing conditions as input data. As a result, it is not possible to derive the suitable manufacturing conditions 13 of the small-diameter short endoscope flexible tube 11SS. The same applies to the reverse. On the other hand, according to the technology of the present disclosure, as described above, the suitable manufacturing condition 13 of the large-diameter long endoscope flexible tube 11LL can be derived by each of the prediction models 90 to 92, as well as the suitable manufacturing condition 13 of the small-diameter short endoscope flexible tube 11SS. That is, it is possible to derive a general-purpose and suitable manufacturing condition 13 regardless of the type of the endoscope flexible tube 11.

As shown in Fig. 15, the learning data 100 of each of the prediction models 90 to 92 is data of each divided portion P, not data of the entire endoscope flexible tube 11. Therefore, a large amount of data can be easily collected. The greater the amount of the learning data 100, the more progress can be made in learning of each of the prediction models 90 to 92, and the prediction accuracy of each of the prediction models 90 to 92 can also be improved. Therefore, the suitable manufacturing condition 13 can be derived with high accuracy as compared with a case in which the quality of the entire endoscope flexible tube 11 is predicted and the suitable manufacturing condition 13 is derived based on the prediction result.

The objective function 67 has the third term 107 and the fourth term 108 that are regularization terms for accommodating the manufacturing conditions of each of the divided portions P in a constraint condition related to the manufacturing condition of the entire endoscope flexible tube 11. Therefore, the suitable manufacturing condition 13 can be accommodated in the constraint condition related to the manufacturing condition of the entire endoscope flexible tube 11.

The third term 107 and the fourth term 108, which are the regularization terms, are terms for smoothing the extrusion amount R per unit time of the resin material of the inner layer 25 of each of the divided portions P and the extrusion amount S per unit time of the resin material of the outer layer 26 of each of the divided portions P. Specifically, the third term 107 is the sum of the second-order differential values of the extrusion amount R per unit time of the resin material of the inner layer 25 of each of the divided portions P, and the fourth term 108 is the sum of the second-order differential values of the extrusion amount S per unit time of the resin material of the outer layer 26 of each of the divided portions P. Therefore, the suitable manufacturing condition 13 can be set as if set by a skilled operator.

The first term 105 and the second term 106, which are the terms including the difference, are the sum of the values obtained by dividing the difference by the elasticity standard value 85 and the total thickness set value 86, which are the target values of the divided portion P. In a case in which the terms including the difference are taken as the sum of the differences without dividing the difference by the target value of the divided portion P, a case in which the target value is 100, the prediction value is 95, and the difference is 5 is treated in the same way as a case in which the target value is 10, the prediction value is 5, and the difference is 5. Therefore, by setting the terms including the difference as the sum of the values obtained by dividing the difference by the target value of the divided portion P, it is possible to separately handle a case in which the target value is 100, the prediction value is 95, and the difference is 5 (the value obtained by dividing the difference by the target value is 5/100 = 0.05) and a case in which the target value is 10, the prediction value is 5, and the difference is 5 (the value obtained by dividing the difference by the target value is 5/10 = 0.5). As a result, the suitable manufacturing condition 13 can be accurately derived.

Each of the prediction models 90 to 92 also receives input of the physical property information 81 of a material constituting the endoscope flexible tube 11 and the design information 82 of the endoscope flexible tube 11. Therefore, it is possible to improve the prediction accuracy of the elasticity prediction value 95, the inner layer thickness prediction value 96, and the outer layer thickness prediction value 97 as compared with a case in which each of the prediction models 90 to 92 does not receive the input of the physical property information 81 and the design information 82.

In addition, in addition to the manufacturing condition 80 of the divided portion P to be predicted, the manufacturing conditions 80 of the divided portions P adjacent to both sides of the divided portion P are input to the elasticity value prediction model 90, so that the prediction accuracy of the elasticity prediction value 95 can be further improved. Then, the manufacturing condition 80A_P1 excluding the information on the outer layer 26 and the like are input to the inner layer thickness prediction model 91, or conversely, the manufacturing condition 80B_P1 excluding the information on the inner layer 25 and the like are input to the outer layer thickness prediction model 92, and the information that is considered to be unnecessary for prediction is excluded, so that the prediction accuracy of the inner layer thickness prediction value 96 and the outer layer thickness prediction value 97 can be further improved.

It is preferable to input both the physical property information 81 and the design information 82 to the elasticity value prediction model 90 or the like, but at least one of the physical property information 81 or the design information 82 may be input to the elasticity value prediction model 90 or the like.

There are a plurality of the prediction values such as the elasticity prediction value 95, the inner layer thickness prediction value 96, and the outer layer thickness prediction value 97, and the machine learning model is prepared for each of the plurality of the prediction values, that is, the elasticity value prediction model 90, the inner layer thickness prediction model 91, and the outer layer thickness prediction model 92. Therefore, all of the elasticity prediction value 95, the inner layer thickness prediction value 96, and the outer layer thickness prediction value 97 can be predicted with high accuracy.

The product is the endoscope flexible tube 11 including the flexible tube substrate 20 and the resin layer 21 that covers the flexible tube substrate 20 and that is composed of the inner layer 25 and the outer layer 26 of which the thickness ratio is different in the axial direction AD. The divided portion P is a portion obtained by dividing the endoscope flexible tube 11 along the axial direction AD. The manufacturing condition 80 includes the extrusion amount per unit time of the resin material of the inner layer 25 and the extrusion amount per unit time of the resin material of the outer layer 26, as obtained from the extrusion molding machine 12. The prediction value includes the elasticity value of the divided portion P (elasticity prediction value 95), the thickness of the inner layer of the divided portion P (inner layer thickness prediction value 96), and the thickness of the outer layer of the divided portion P (outer layer thickness prediction value 97).

There are a wide variety of types of the endoscope flexible tube 11, such as the large-diameter long endoscope flexible tube 11LL of an oral endoscope for an upper digestive tract shown in Fig. 22 or the small-diameter short endoscope flexible tube 11SS of a transnasal endoscope for bronchi shown in Fig. 23. In addition, specification changes, such as changes in the resin materials of the inner layer 25 and the outer layer 26 and changes in the outer diameter of the flexible tube substrate 20, are also frequent. Therefore, it is possible to fully exhibit the effect of deriving the general-purpose and suitable manufacturing condition 13 regardless of the type of the endoscope flexible tube 11.

The controller 41 of the extrusion molding machine 12 may function as the CPU 57 of the information processing apparatus 10. That is, the output of each of the prediction values 95 to 98 and the derivation of the suitable manufacturing condition 13 may be performed by the controller 41 of the extrusion molding machine 12. In this case, the extrusion molding machine 12 itself is an example of an "information processing apparatus" according to the technology of the present disclosure.

### [Second Embodiment]

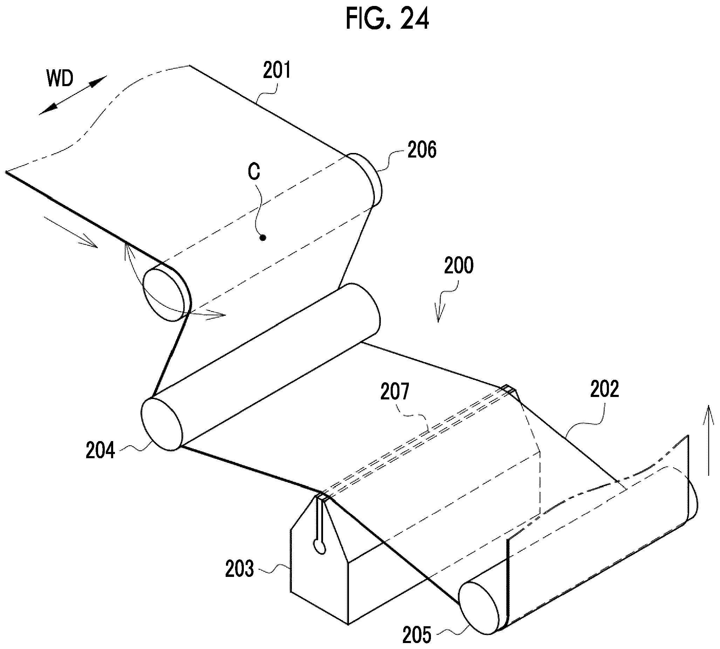

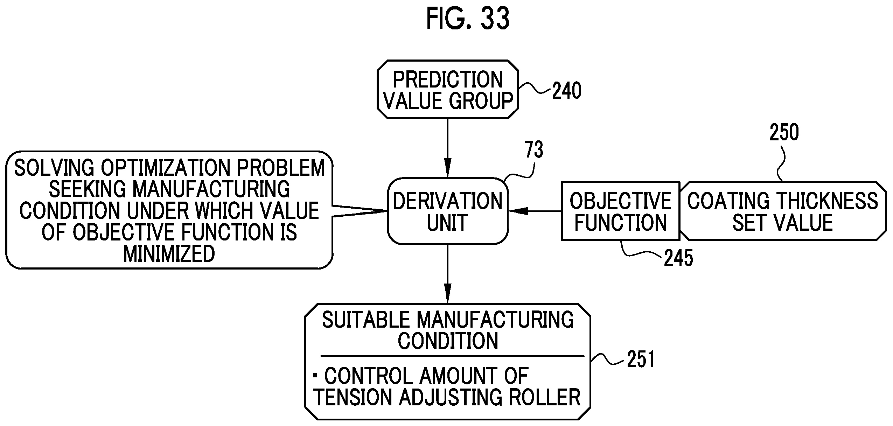

In the first embodiment, the flexible endoscope flexible tube 11 is exemplified as a product, and an example in which the suitable manufacturing condition 13 of the extrusion molding machine 12 is derived has been described, but the technology of the present disclosure is not limited to this. In a second embodiment, a suitable manufacturing condition 251 (see Fig. 33) of a sheet manufacturing apparatus 200 (see Fig. 24) is derived.

As shown in Fig. 24 as an example, the sheet manufacturing apparatus 200 is an apparatus that applies a liquid to a long support 201 to be transported to manufacture a sheet 202. The sheet 202 is an example of a "product" according to the technology of the present disclosure. The support 201 is, for example, a substrate of a magnetic tape. In this case, the liquid is a magnetic layer material, and the sheet 202 is a magnetic tape. Alternatively, the support 201 is, for example, a substrate of an optical film. In this case, the liquid is a photosensitive layer material, and the sheet 202 is an optical film.

The sheet manufacturing apparatus 200 includes a coating unit 203, transport rollers 204 and 205, and a tension adjusting roller 206. The coating unit 203 is a geeser or the like for applying a liquid to the support 201. The transport rollers 204 and 205 are disposed at symmetrical positions on an upstream side and a downstream side with the coating unit 203 interposed therebetween, and transport the support 201 and the sheet 202 by being rotated by a motor. The tension adjusting roller 206 is disposed on the upstream side of the coating unit 203 and the transport roller 204. The tension adjusting roller 206 adjusts a tension applied to the support 201 at an application location of the liquid of the coating unit 203 by tilting, for example, with respect to the support 201 and the sheet 202 in a width direction WD with a center C as a rotation center, as indicated by an arrow. The application location is a slit 207 having a narrow gap parallel to the width direction WD. The sheet manufacturing apparatus 200 also includes a delivery drum that delivers the roll-shaped support 201 toward the coating unit 203, a winding drum that winds the sheet 202 coated with the liquid from the coating unit 203, and the like. In addition, the sheet manufacturing apparatus 200 also includes a driven roller that comes into contact with the support 201 or the sheet 202 and that is rotationally driven in response to the transport of the support 201 or the sheet 202, a dancer roller that suppresses fluctuations in the tension applied to the support 201 by swinging in accordance with the change in the transportation speed of the support 201, and the like.

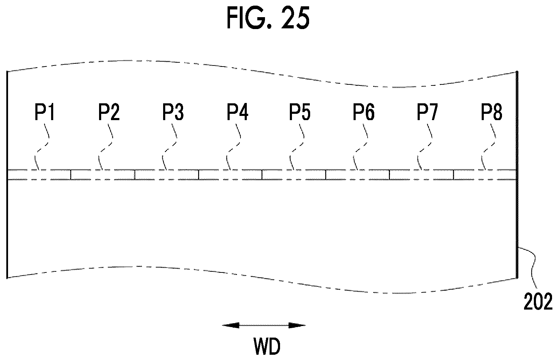

As shown in Fig. 25 as an example, in the second embodiment, quality of each of divided portions P1, P2, ..., P7, and P8 divided along the width direction WD of the sheet 202 is predicted.

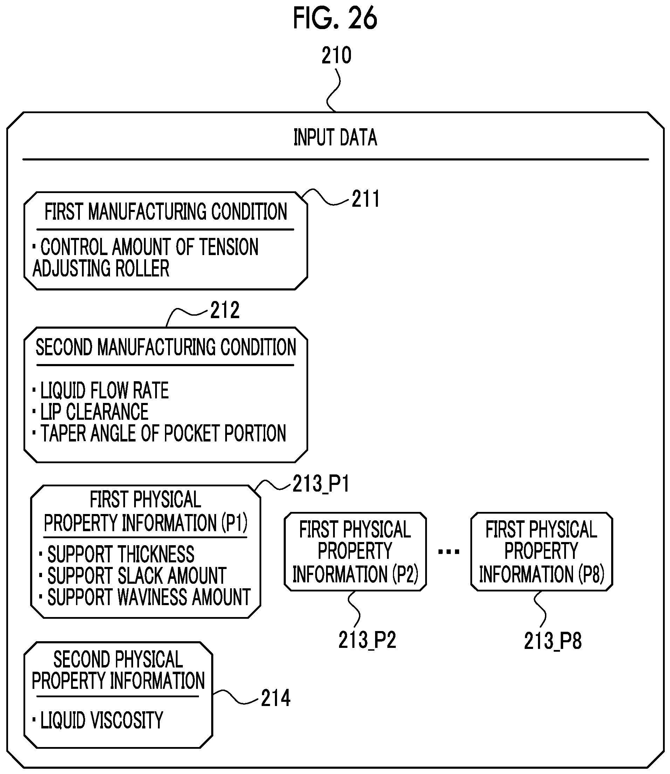

As shown in Fig. 26 as an example, input data 210 of the second embodiment includes a first manufacturing condition 211 and a second manufacturing condition 212. The first manufacturing condition 211 includes a control amount of the tension adjusting roller 206. The control amount of the tension adjusting roller 206 is, in other words, a pushing amount of the tension adjusting roller 206 in the width direction WD. The second manufacturing condition 212 includes a liquid flow rate from the coating unit 203, a lip clearance, and a taper angle of a pocket portion. The lip clearance is a gap between the slit 207, which is the application location, and the support 201. The pocket portion is provided in the coating unit 203 and communicates with the slit 207. The pocket portion is a portion for spreading the liquid in the width direction WD. In addition, as the first manufacturing condition 211, a swing amount of the dancer roller and the like may be appropriately added.

The input data 210 includes first physical property information 213_P1 of the divided portion P1, first physical property information 213_P2 of the divided portion P2, ..., and first physical property information 213_P8 of the divided portion P8. In addition, the input data 210 includes second physical property information 214. Hereinafter, in a case in which there is no need to particularly distinguish between the first physical property information 213_P1 to the first physical property information 213_P8 of the divided portions P1 to P8, the first physical property information 213_P1 to the first physical property information 213_P8 may be referred to as first physical property information 213.

The first physical property information 213 and the second physical property information 214 are information related to the physical properties of a material constituting the sheet 202, here, the support 201 and the liquid. The first physical property information 213 includes a thickness, an amount of slack, and an amount of waviness of the support 201 in the divided portion P. The thickness, the amount of slack, and the amount of waviness of the support 201 are values measured for each divided portion P by a sensor (not shown) installed between the coating unit 203 and the transport roller 204. Therefore, the first physical property information 213 changes from moment to moment as the support 201 is transported. Therefore, in the second embodiment, the input data 210 is input each time the support 201 is transported by a preset length. The preset length is, for example, 5 cm to 10 cm. The thickness, the amount of slack, and the amount of waviness of the support 201 are representative values at a preset length, for example, an average value. The second physical property information 214 includes a liquid viscosity (for example, an elongation viscosity). The first manufacturing condition 211, the second manufacturing condition 212, the first physical property information 213, and the second physical property information 214 are not limited to the exemplified contents, and other elements may be appropriately added.

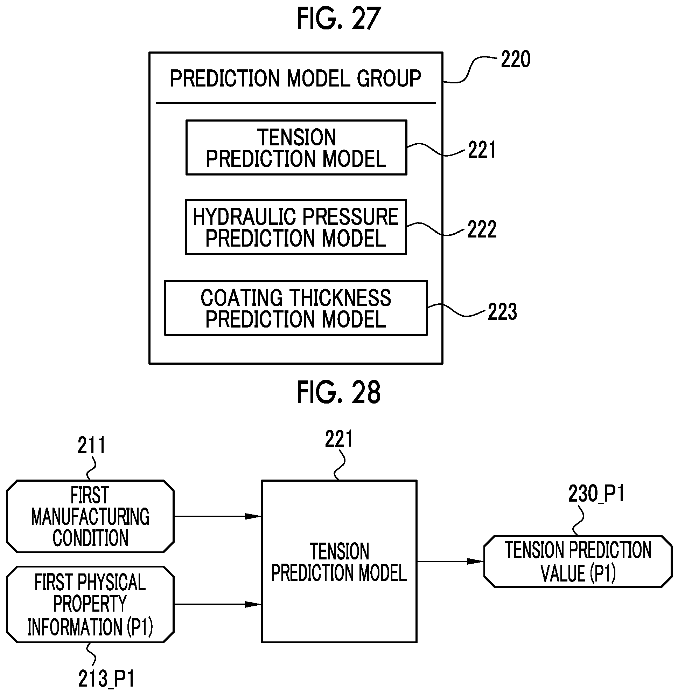

As shown in Fig. 27 as an example, a prediction model group 220 of the second embodiment includes a tension prediction model 221, a hydraulic pressure prediction model 222, and a coating thickness prediction model 223. The tension prediction model 221, the hydraulic pressure prediction model 222, and the coating thickness prediction model 223 are machine learning models configured by, for example, a neural network. That is, the tension prediction model 221, the hydraulic pressure prediction model 222, and the coating thickness prediction model 223 are examples of a "machine learning model" according to the technology of the present disclosure.

The tension prediction model 221 outputs a prediction value of the tension applied to the divided portion P in the slit 207, which is the application location, (hereinafter, referred to as a tension prediction value) in response to the input of the first manufacturing condition 211 and the first physical property information 213. The hydraulic pressure prediction model 222 outputs a prediction value of the pressure of the liquid applied to the divided portion P in the slit 207 (hereinafter, referred to as a hydraulic pressure prediction value) in response to the input of the second manufacturing condition 212, the first physical property information 213, and the second physical property information 214. The coating thickness prediction model 223 outputs a prediction value of the coating thickness of the liquid on the divided portion P (hereinafter, referred to as a coating thickness prediction value) in response to the input of the tension prediction value and the hydraulic pressure prediction value. As described above, in the second embodiment as well, there are a plurality of types of the prediction values, and a prediction model is prepared for each of the plurality of types of the prediction values.

As shown in Fig. 28 as an example, in a case of predicting the quality of the divided portion P1, the prediction unit 72 inputs the first manufacturing condition 211 and the first physical property information 213_P1 of the divided portion P1 to the tension prediction model 221. Then, the prediction unit 72 outputs a tension prediction value 230_P1 of the divided portion P1 from the tension prediction model 221.

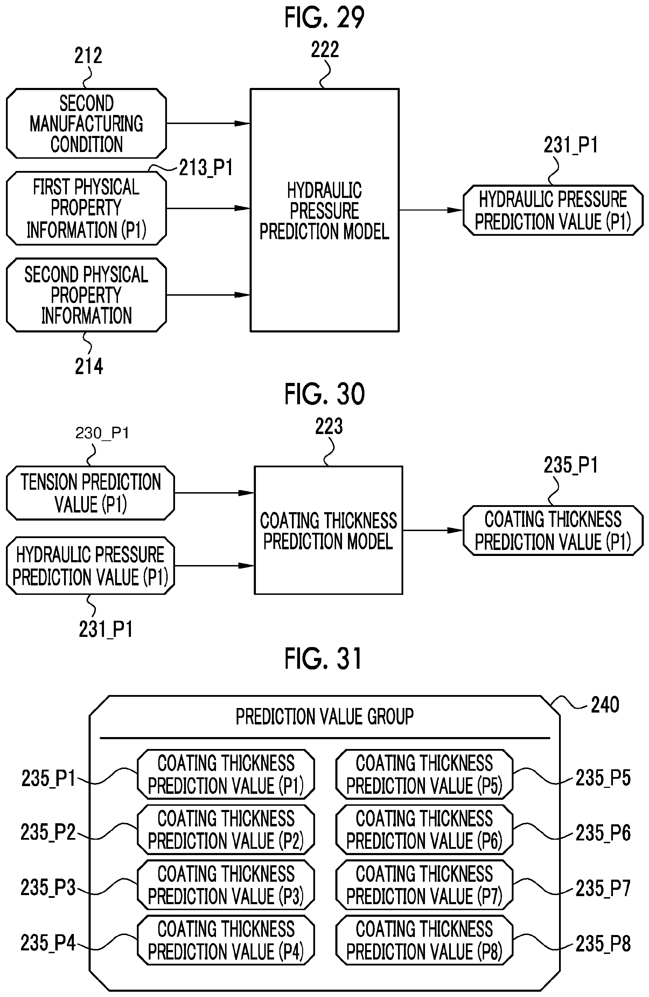

As shown in Fig. 29 as an example, in a case of predicting the quality of the divided portion P1, the prediction unit 72 inputs the second manufacturing condition 212, the first physical property information 213_P1 of the divided portion P1, and the second physical property information 214 to the hydraulic pressure prediction model 222. Then, the prediction unit 72 outputs a hydraulic pressure prediction value 231_P1 of the divided portion P1 from the hydraulic pressure prediction model 222.

As shown in Fig. 30 as an example, in a case of predicting the quality of the divided portion P1, the prediction unit 72 inputs the tension prediction value 230_P1 output from the tension prediction model 221 and the hydraulic pressure prediction value 231_P1 output from the hydraulic pressure prediction model 222 to the coating thickness prediction model 223. Then, the prediction unit 72 outputs a coating thickness prediction value 235_P1 of the divided portion P1 from the coating thickness prediction model 223.

In Figs. 28 to 30, an example has been described in which the tension prediction value 230_P1, the hydraulic pressure prediction value 231_P1, and the coating thickness prediction value 235_P1 of the divided portion P1 are output from the prediction models 221 to 223, but, similarly for the remaining divided portions P2 to P8, the prediction unit 72 outputs tension prediction values 230_P2 to 230_P8 (not shown), hydraulic pressure prediction values 231_P2 to 231_P8 (not shown), and coating thickness prediction values 235_P2 to 235_P8 (see Fig. 31) from the prediction models 221 to 223, respectively. Hereinafter, in a case in which there is no need to particularly distinguish between the coating thickness prediction values 235_P1 to 235_P8 of the divided portions P1 to P8, the coating thickness prediction values 235_P1 to 235_P8 may be referred to as an coating thickness prediction value 235.

By obtaining the coating thickness prediction value 235 of each divided portion P in this way, the prediction value group 240 of the second embodiment has a content as shown in Fig. 31 as an example. That is, the prediction value group 240 includes the coating thickness prediction value 235_P1 of the divided portion P1, the coating thickness prediction value 235_P2 of the divided portion P2, ..., the coating thickness prediction value 235_P7 of the divided portion P7, and the coating thickness prediction value 235_P8 of the divided portion P8.

The learning data of each of the prediction models 221 to 223 is data collected from the divided portion P of the sheet 202 manufactured in the past. The learning data of each of the prediction models 221 to 223 is not shown, but is as follows. That is, the input data for learning includes a first manufacturing condition for learning corresponding to the first manufacturing condition 211, a second manufacturing condition for learning corresponding to the second manufacturing condition 212, a first physical property information for learning corresponding to the first physical property information 213, and a second physical property information for learning corresponding to the second physical property information 214. Then, the correct answer data includes an actual measured value of the tension applied to the divided portion P in the slit 207, an actual measured value of the pressure of the liquid applied to the divided portion P in the slit 207, and an actual measured value of the coating thickness of the liquid on the divided portion P.

In addition, the learning data of each of the prediction models 221 to 223 is data collected from various types of sheets 202 manufactured in the past, such as a case of a support 201 having a relatively large width dimension and the number of the divided portions P being 10 or more and a case of a support 201 having a relatively small width dimension and the number of the divided portions P being less than five.

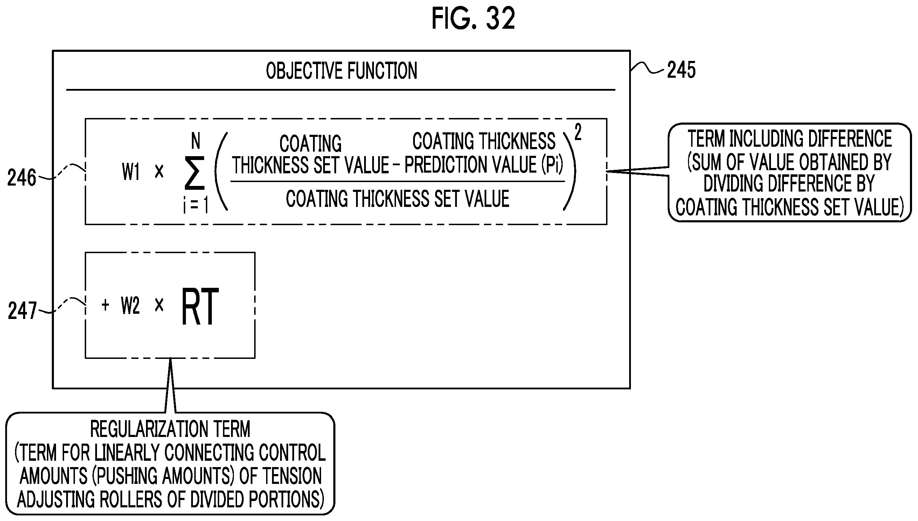

As shown in Fig. 32 as an example, an objective function 245 of the second embodiment has a first term 246 and a second term 247. The first term 246 includes a difference between the coating thickness prediction value 235 and a coating thickness set value 250 (see Fig. 33) of each of the divided portions P. More specifically, the first term 246 includes a sum of squares of a value obtained by dividing the difference between the coating thickness prediction value 235 and the coating thickness set value 250 by the coating thickness set value 250. The first term 246 is a term obtained by multiplying the sum by a first weight coefficient W1. Since the coating thickness set value 250 is common to the respective divided portions P, there is only one coating thickness set value 250. The first term 246 is an example of a "term including a difference" according to the technology of the present disclosure. In addition, the coating thickness set value 250 is an example of a "target value" according to the technology of the present disclosure.

The second term 247 is a regularization terms for accommodating the manufacturing condition of each of the divided portions P in a constraint condition related to the manufacturing condition of the entire sheet 202. In the constraint condition related to the manufacturing condition of the entire sheet 202, here, the control amounts (the pushing amounts of the tension adjusting rollers 206) of the tension adjusting rollers 206 of the divided portions P are linearly connected. Therefore, the second term 247 is a term for linearly connecting the control amounts (pushing amounts) of the tension adjusting rollers 206 of the divided portions P. The second term 247 is, for example, 1 minus a coefficient of decision in a case in which the pushing amount of the tension adjusting roller 206 of each of the divided portions P is subjected to linear regression analysis. The second term 247 has a second weight coefficient W2.

The first weight coefficient W1 and the second weight coefficient W2 are values (W1 + W2 = 1) of which the total is 1, similarly to the first weight coefficient W1 to the fourth weight coefficient W4 of the first embodiment. For example, W1 = W2 = 0.5. The first weight coefficient W1 and the second weight coefficient W2 do not necessarily have the same value. For example, in a case in which it is desired to place importance on the first term 246, that is, in a case in which it is desired to bring the coating thickness prediction value 235 of each of the divided portions P closer to the coating thickness set value 250, the first weight coefficient W1 may be set to a value higher than the second weight coefficient W2.

As shown in Fig. 33 as an example, the suitable manufacturing condition 251 of the second embodiment derived by the derivation unit 73 by solving an optimization problem seeking a manufacturing condition under which the value of the objective function 245 is minimized includes the control amount of the tension adjusting roller 206. As for the control amount of the tension adjusting roller 206, one value common to each divided portion P is derived by the function of the second term 247 of the objective function 245.

In the second embodiment, as described above, the input data 210 is input each time the support 201 is transported by a preset length. Therefore, the prediction of the tension prediction values 230_P1 to 230_P8, the hydraulic pressure prediction values 231_P1 to 231_P8, and the coating thickness prediction value 235 by means of the prediction unit 72 and the derivation of the control amount of the tension adjusting roller 206 as the suitable manufacturing condition 251 by means of the derivation unit 73 are also performed each time the support 201 is transported by a preset length and the input data 210 is input. In this way, by continuously deriving the control amount of the tension adjusting roller 206 as the suitable manufacturing condition 251, the liquid can be applied to the support 201 in a state in which a suitable tension is always applied from the tension adjusting roller 206 to the support 201 with respect to the continuously transported support 201.

As described above, in the second embodiment, the product is the sheet 202 obtained by applying the liquid to the long support 201 to be transported. The divided portion P is a portion obtained by dividing the sheet 202 along the width direction WD. The first manufacturing condition 211 includes the control amount of the tension adjusting roller 206 that is disposed on the upstream side of the slit 207, which is the application location of the liquid, and that adjusts the tension applied to the support 201 in the slit 207. The prediction value includes the coating thickness (coating thickness prediction value 235) of the liquid in the divided portion P. Therefore, it is possible to handle various types such as a case of a support 201 having a relatively large width dimension and the number of the divided portions P being 10 or more, and a case of a support 201 having a relatively small width dimension and the number of the divided portions P being less than five. That is, it is possible to derive a general-purpose and suitable manufacturing condition 251 regardless of the type of the sheet 202. The prediction value of the tension applied to the divided portion P in the slit 207 and the prediction value of the hydraulic pressure applied to the divided portion P in the slit 207 are derived, and then the coating thickness prediction value of the liquid of the divided portion P is derived, but the present invention is not limited to this. The coating thickness prediction value may be derived without deriving the tension prediction value and the hydraulic pressure prediction value based on the first manufacturing condition 211 and the like.

### [Third Embodiment]

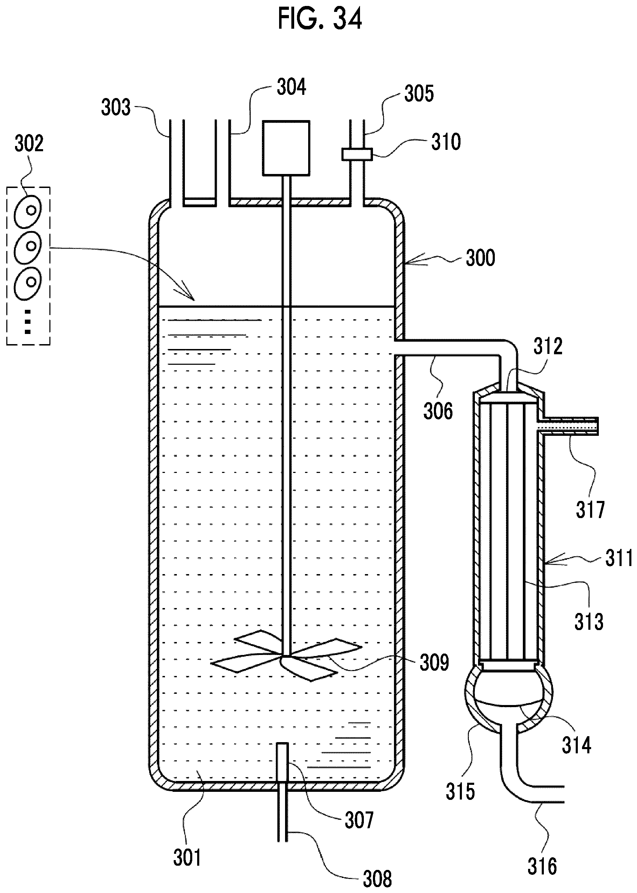

In a third embodiment, a suitable manufacturing condition in a culture tank 300 (see Fig. 34) is derived.

As shown in Fig. 34 as an example, a culture solution 301 is stored in the culture tank 300. The accommodation capacity of the culture tank 300 is, for example, 50 L or more and 5000 L or less. Cells 302 are seeded in the culture tank 300, and the cells 302 are cultured in the culture solution 301. The cells 302 are an example of a "product" according to the technology of the present disclosure.

The cells 302 are, for example, antibody-producing cells established by incorporating an antibody gene into a cell such as Chinese Hamster Ovary cell. The cells 302 produce an immunoglobulin, that is, an antibody, as a product in the culture process. Therefore, not only the cells 302 but also the antibody are present in the culture solution 301. The antibody is, for example, a monoclonal antibody and serves as an active ingredient of a biopharmaceutical.

The culture tank 300 is provided with a culture medium supply path 303, a gas supply path 304, an exhaust path 305, a culture solution delivery/recovery path 306, a sparger 307, a gas supply path 308, a stirring blade 309, and the like. The culture medium supply path 303 is a flow passage for continuously supplying a fresh culture medium into the culture tank 300. That is, perfusion culture is performed in the culture tank 300. The gas supply path 304 is a flow passage for supplying a gas containing air and carbon dioxide from the upper part. The exhaust path 305 is a flow passage for exhausting the gas supplied from the gas supply path 304 to an outside of the culture tank 300. An exhaust filter 310 is provided in the exhaust path 305.

The culture solution delivery/recovery path 306 is connected to an inlet/outlet 312 of a cell separation filter 311. The culture solution delivery/recovery path 306 is a flow passage for delivering the culture solution 301 in the culture tank 300 to the cell separation filter 311. In addition, the culture solution delivery/recovery path 306 is a flow passage for returning the culture solution 301 (concentrated solution) from the cell separation filter 311 into the culture tank 300.

The sparger 307 is disposed at a bottom of the culture tank 300. The sparger 307 releases a gas containing oxygen, which is supplied from the gas supply path 308, into the culture tank 300. The oxygen released from the sparger 307 is dissolved in the culture solution 301 and supports the antibody production activity of the cells 302. The stirring blade 309 is rotated at a predetermined rotation speed by a motor or the like to stir the culture solution 301 in the culture tank 300. As a result, homogeneity of the culture solution 301 in the culture tank 300 is maintained. In addition to these members, the culture tank 300 is provided with a flow passage for a cell breeding treatment of intentionally taking out a part of the culture solution 301. The stirring blade 309 may have a plurality of blades as shown in the drawing or may have one disk-shaped blade, and the shape thereof is not particularly limited. In addition, two or more stirring blades 309 may be disposed in the culture tank 300.

The cell separation filter 311 connected to the culture solution delivery/recovery path 306 has a filter membrane 313 inside. The filter membrane 313 captures the cells 302 and transmits the antibody. With the cell separation filter 311, a culture supernatant liquid is obtained by, for example, Tangential Flow Filtration (TFF) method in which the cells 302 are removed from the culture solution 301 by the filter membrane 313.

More specifically, the cell separation filter 311 has a diaphragm pump 315 having an elastic film 314 inside, and a degassing/gas suppling path 316. Air on the lower side of the elastic film 314 is degassed from the degassing/gas suppling path 316, and the elastic film 314 is elastically deformed to be attached to a lower end of the diaphragm pump 315, whereby the culture solution 301 in the culture tank 300 flows into the cell separation filter 311 via the culture solution delivery/recovery path 306. In addition, the culture solution 301 (concentrated solution) which could not pass through the filter membrane 313 is returned to the culture tank 300 via the culture solution delivery/recovery path 306, by supplying air from the degassing/gas suppling path 316 to the lower side of the elastic film 314 and elastically deforming the elastic film 314 to be attached to the upper side of the diaphragm pump 315.

The culture supernatant liquid flows out from an outlet 317 of the cell separation filter 311. The culture supernatant liquid mainly contains an antibody. The culture supernatant liquid is sent to a downstream purification section (not shown), and is subjected to various chromatography treatments, a virus inactivation treatment, and the like in the purification section, and is ultimately made into an active pharmaceutical ingredient of a biopharmaceutical.

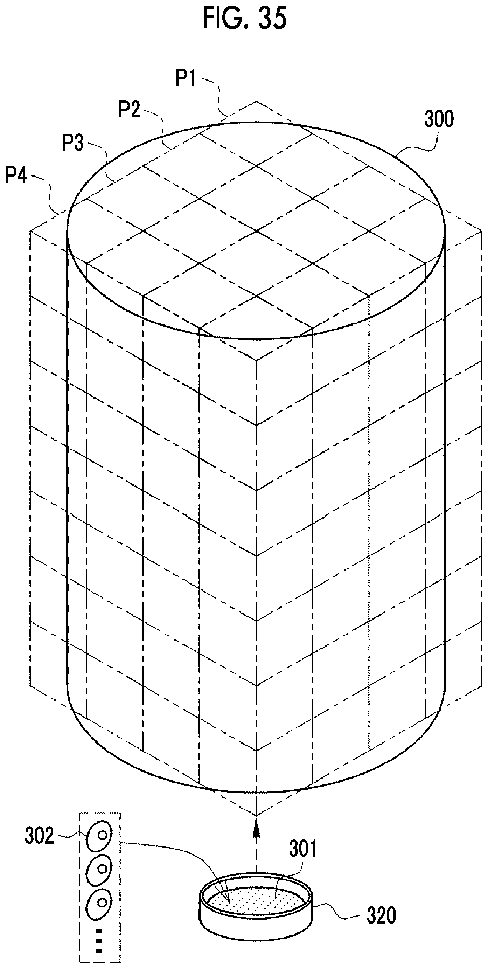

As shown in Fig. 35 as an example, in the third embodiment, quality of each of cubic divided portions P1, P2, P3, P4, ..., which are obtained by dividing the culture tank 300 three-dimensionally, is predicted. Here, one divided portion P is a portion corresponding to a culture container 320 having a capacity of, for example, several mL to several L, such as a petri dish having a smaller scale than the culture tank 300.

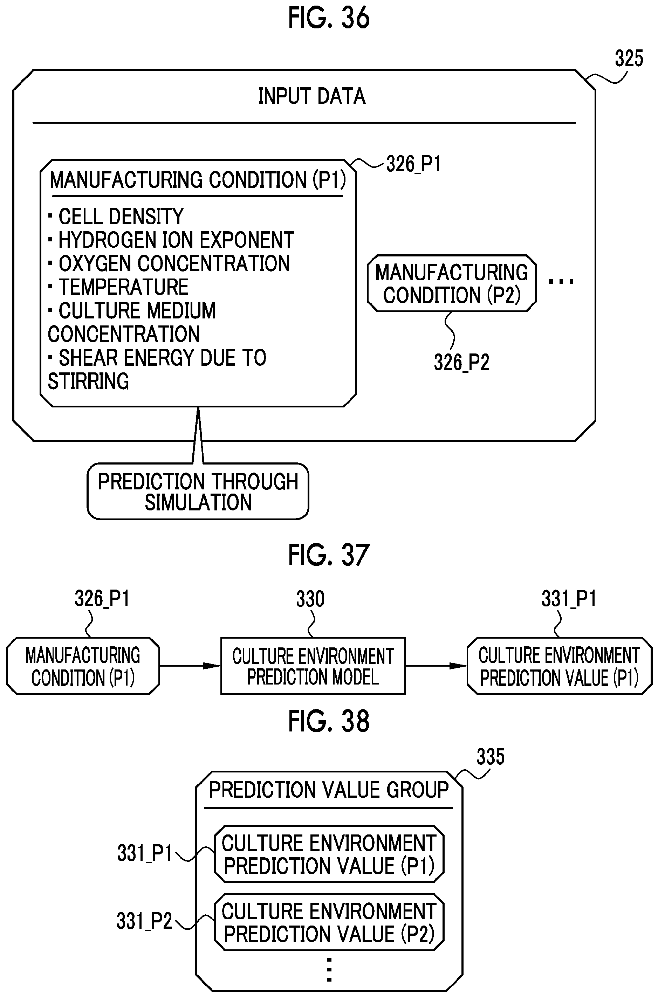

As shown in Fig. 36 as an example, input data 325 of the third embodiment includes a manufacturing condition 326_P1 of the divided portion P1, a manufacturing condition 326_P2 of the divided portion P2, and the like. Hereinafter, in a case in which there is no need to particularly distinguish between the manufacturing condition 326_P1 of the divided portion P1, the manufacturing condition 326_P2 of the divided portion P2, and the like, the manufacturing condition 326_P1 of the divided portion P1, the manufacturing condition 326_P2 of the divided portion P2, and the like may be referred to as a manufacturing condition 326.

The manufacturing condition 326 includes a culture condition of the cells 302. Specifically, the manufacturing condition 326 includes a density (cell density) of the cells 302 in the divided portion P, and a hydrogen ion exponent, an oxygen concentration, a temperature, a culture medium concentration, and shear energy due to stirring with the stirring blade 309 in the divided portion P. All of these are values predicted by simulation using various parameters such as a capacity of the culture tank 300, a position of the culture medium supply path 303, a position of the gas supply path 304, a position of the sparger 307, a position of the stirring blade 309, a supply amount of the medium, a supply amount of the gas, and a rotation speed of the stirring blade 309. In addition, a carbon dioxide concentration and the like may be appropriately added as the manufacturing condition 326. In addition, physical property information of the cell 302, such as a size of the cell 302, and/or design information, such as the capacity of the culture tank 300, may be added to the input data 325.

As shown in Fig. 37 as an example, in the third embodiment, a culture environment prediction model 330 is used. The culture environment prediction model 330 is a machine learning model configured by, for example, a neural network. That is, the culture environment prediction model 330 is an example of a "machine learning model" according to the technology of the present disclosure.

In a case of predicting the quality of the divided portion P1, the prediction unit 72 inputs the manufacturing condition 326_P1 to the culture environment prediction model 330. Then, the prediction unit 72 outputs a culture environment prediction value 331_P1 of the divided portion P1 from the culture environment prediction model 330.

In Fig. 37, an example has been described in which the culture environment prediction value 331_P1 of the divided portion P1 is output from the culture environment prediction model 330, but, similarly for the remaining divided portions P2 and the like, the prediction unit 72 outputs culture environment prediction value 331_P2 (see Fig. 38) and the like from the culture environment prediction model 330. Hereinafter, in a case in which there is no need to particularly distinguish between the culture environment prediction values 331_P1, 331_P2, and the like of the divided portions P1, P2, and the like, the culture environment prediction values 331_P1, 331_P2, and the like may be referred to as a culture environment prediction value 331.

The culture environment prediction value 331 is a value representing the goodness or badness of the culture environment for the cell 302 based on a proliferation rate, a survival rate, and the like of the cell 302, and takes, for example, a value between 0 and 100. In a case in which the value is close to 100, the culture environment for the cell 302 is good. The culture environment prediction value 331 is an example of an "index value" according to the technology of the present disclosure.

By obtaining the culture environment prediction value 331 of each divided portion P in this way, the prediction value group 335 of the third embodiment has a content as shown in Fig. 38 as an example. That is, the prediction value group 335 includes the culture environment prediction value 331_P1 of the divided portion P1, the culture environment prediction value 331_P2 of the divided portion P2, and the like.

The learning data of the culture environment prediction model 330 is data collected from cells 302 produced in the past using the small-scale culture container 320. The learning data of the culture environment prediction model 330 is not shown, but is as follows. That is, a manufacturing condition for learning corresponding to the manufacturing condition 326 is included as the input data for learning. Then, the correct answer data includes an actual measured value of the goodness or badness of the culture environment for the cell 302 in the small-scale culture container 320.

In addition, the learning data of the culture environment prediction model 330 is data collected from cells 302 of various aspects produced in the past using small-scale culture container 320, such as a case of cells producing an antibody A and a case of cells producing an antibody B, or a case of culture in the culture container 320 having a capacity of 50 mL and a case of culture in the culture container 320 having a capacity of 1 L.

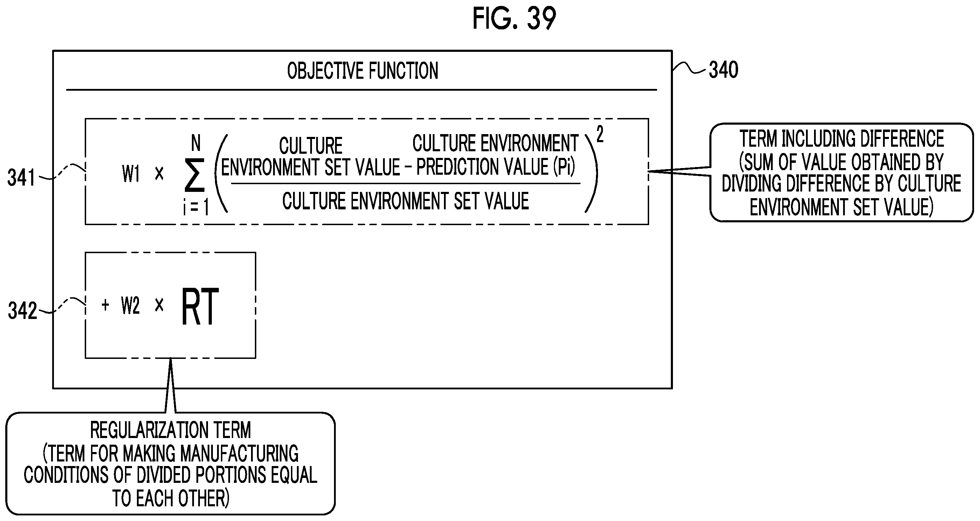

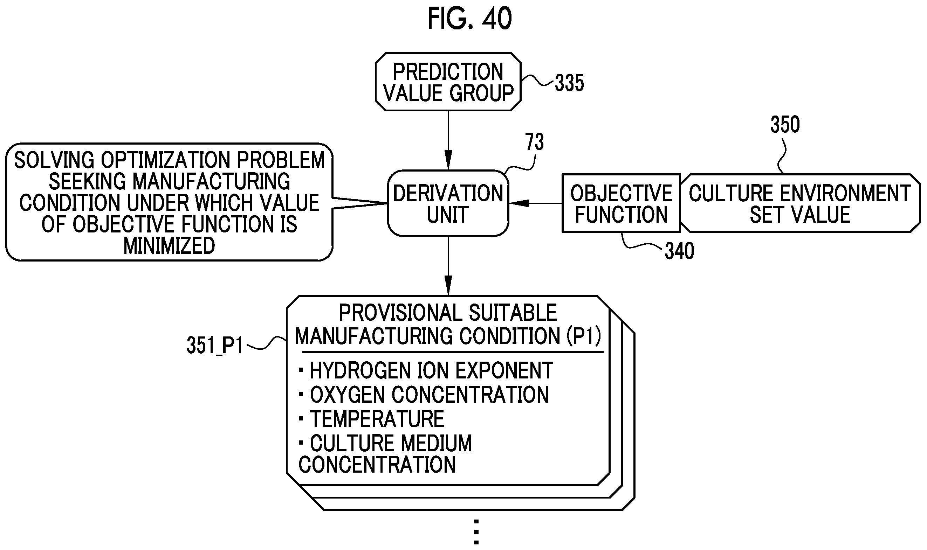

As shown in Fig. 39 as an example, an objective function 340 of the third embodiment has a first term 341 and a second term 342. The first term 341 includes a difference between the culture environment prediction value 331 and a culture environment set value 350 (see Fig. 40) of each of the divided portions P. More specifically, the first term 341 includes a sum of squares of a value obtained by dividing the difference between the culture environment prediction value 331 and the culture environment set value 350 by the culture environment set value 350. The first term 341 is a term obtained by multiplying the sum by a first weight coefficient W1. Since the culture environment set value 350 is common to the respective divided portions P, there is only one culture environment set value 350. The first term 341 is an example of a "term including a difference" according to the technology of the present disclosure. In addition, the culture environment set value 350 is an example of a "target value" according to the technology of the present disclosure.

The second term 342 is a regularization terms for accommodating the manufacturing condition of each of the divided portions P in a constraint condition related to the manufacturing condition of the entire cells 302 in the culture tank 300. In the constraint condition related to the manufacturing condition of the entire cells 302 in the culture tank 300, the manufacturing conditions of the divided portions P are made to be equal to each other. Therefore, the second term 342 is a term for making the manufacturing conditions of the divided portions P equal to each other. The second term 342 is, for example, a variance or a standard deviation of the manufacturing conditions of each of the divided portions P. In this case, in a case in which the variance or the standard deviation is minimized, the manufacturing conditions of the divided portions P can be made to be equal to each other. The term "equal" includes not only a case in which the manufacturing conditions of the divided portions P are completely the same but also a case in which the manufacturing conditions of the divided portions P fall within a preset difference range. The second term 342 has a second weight coefficient W2.

The first weight coefficient W1 and the second weight coefficient W2 are values (W1 + W2 = 1) of which the total is 1, similarly to the first weight coefficient W1 to the fourth weight coefficient W4 of the first embodiment. For example, W1 = W2 = 0.5. The first weight coefficient W1 and the second weight coefficient W2 do not necessarily have the same value. For example, in a case in which it is desired to place importance on the first term 341, that is, in a case in which it is desired to bring the culture environment prediction value 331 of each of the divided portions P closer to the culture environment set value 350, the first weight coefficient W1 may be set to a value higher than the second weight coefficient W2.

As shown in Fig. 40 as an example, in the third embodiment, the derivation unit 73 derives a provisional suitable manufacturing condition 351 of each of the divided portions P, such as a provisional suitable manufacturing condition 351_P1 of the divided portion P1 by solving an optimization problem seeking a manufacturing condition under which the value of the objective function 340 is minimized. The provisional suitable manufacturing condition 351 includes a hydrogen ion exponent, an oxygen concentration, a temperature, and a culture medium concentration in the divided portion P. Here, it is assumed that the hydrogen ion exponent, the oxygen concentration, the temperature, and the culture medium concentration are almost uniform in each divided portion P due to the stirring of the stirring blade 309. On the other hand, it is assumed that the shear energy due to the stirring of the stirring blade 309 is different for each divided portion P.

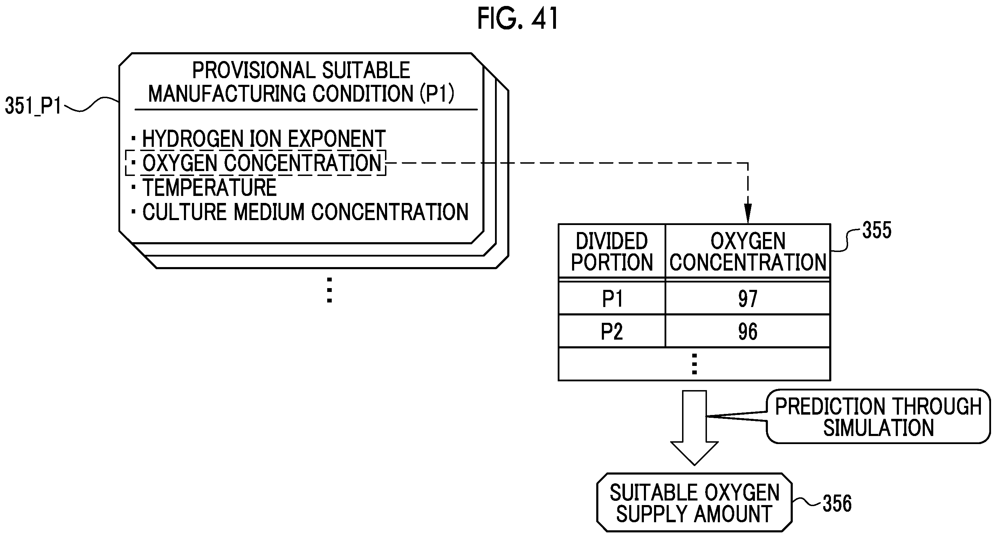

As shown in Fig. 41 as an example, the derivation unit 73 further derives a more suitable manufacturing condition from the provisional suitable manufacturing condition 351 of each of the divided portions P. In Fig. 41, as shown in Table 355, a state is shown in which a suitable oxygen supply amount 356 to be supplied into the culture tank 300 via the sparger 307 is derived from the oxygen concentration of the provisional suitable manufacturing condition 351 of each of the divided portions P. The derivation unit 73 derives the suitable oxygen supply amount 356 by predicting, through simulation, an oxygen supply amount such that the oxygen concentration of each of the divided portions P is the oxygen concentration of the provisional suitable manufacturing condition 351. The suitable oxygen supply amount 356 is an example of a "suitable manufacturing condition" according to the technology of the present disclosure. In addition to or instead of the suitable oxygen supply amount 356, a suitable culture medium supply amount from the culture medium supply path 303, a suitable gas supply amount from the gas supply path 304, a suitable rotation speed of the stirring blade 309, or the like may be derived as the suitable manufacturing condition.

As described above, in the third embodiment, the product is the cells 302 cultured in the culture tank 300. The divided portion P is a portion obtained by dividing the culture tank 300. The manufacturing condition 326 includes a culture condition of the cell 302, and the prediction value includes an index value (culture environment prediction value 331) indicating desirability of the culture environment for the cell 302. Therefore, it is possible to handle various aspects such as a case of a cell that produces an antibody A and a case of a cell that produces an antibody B. That is, it is possible to derive a general-purpose and suitable manufacturing condition regardless of the type of the cell 302.

The cell 302 is not limited to the antibody-producing cell as exemplified. The cell 302 may be a pluripotent stem cell such as an induced pluripotent stem (iPS) cell. As the machine learning model, a plurality of types of models, such as a model that predicts the proliferation rate of the cell 302 of each divided portion P and a model that predicts the survival rate of the cell 302 of each divided portion P, may be prepared.

The information processing apparatus 10 may be installed in the same facility as the extrusion molding machine 12 and the like, or may be installed in a data center independent of the facility in which the extrusion molding machine 12 and the like are installed.

In each of the above embodiments, for example, as hardware structures of processing units that execute various kinds of processing, such as the reception unit 70, the RW controller 71, the prediction unit 72, the derivation unit 73, and the distribution controller 74, various processors shown below can be used. As described above, the various processors include, in addition to the CPU 57 that is a general-purpose processor which executes software (operation program 65) to function as various processing units, a programmable logic device (PLD) that is a processor of which a circuit configuration can be changed after manufacture, such as a field programmable gate array (FPGA), and a dedicated electrical circuit that is a processor having a circuit configuration which is designed for exclusive use to execute specific processing, such as an application specific integrated circuit (ASIC).

One processing unit may be configured of one of the various processors or may be configured of a combination of two or more processors of the same type or different types (for example, a combination of a plurality of FPGAs and/or a combination of a CPU and an FPGA). In addition, a plurality of processing units may be configured of one processor.

As an example of configuring the plurality of processing units with one processor, first, there is a form in which, as typified by computers such as a client and a server, one processor is configured of a combination of one or more CPUs and software and the processor functions as the plurality of processing units. Second, there is a form in which, as typified by a system on chip (SoC) and the like, a processor that implements functions of an entire system including the plurality of processing units with one integrated circuit (IC) chip is used. As described above, various processing units are configured by using one or more of the various processors as a hardware structure.

In addition, more specifically, an electric circuit (circuitry) in which circuit elements, such as semiconductor elements, are combined can be used as the hardware structure of these various processors.

It is possible to understand the technologies described in following Appendices from the above description.
[Appendix 1] An information processing apparatus comprising:
   a processor,
   in which the processor
      uses a machine learning model that outputs a prediction value of quality of a divided portion obtained by dividing an entire product in response to input of a manufacturing condition of the divided portion,
      inputs the manufacturing condition of the divided portion to the machine learning model and outputs the prediction value of the divided portion from the machine learning model, and
      derives a suitable manufacturing condition under which quality of the entire product reaches a target value by solving an optimization problem seeking a manufacturing condition under which a value of an objective function having a term including a difference between the prediction value of the divided portion and a target value of the quality of the divided portion is minimized.
[Appendix 2] The information processing apparatus according to Appendix 1,
   in which the objective function has a regularization term for accommodating the manufacturing condition of the divided portion in a constraint condition related to a manufacturing condition of the entire product.
[Appendix 3] The information processing apparatus according to Appendix 2,
   in which the regularization term is a term for smoothing the manufacturing condition of the divided portion.
[Appendix 4] The information processing apparatus according to Appendix 3,
   in which the regularization term is a sum of second-order differential values of the manufacturing condition of the divided portion.
[Appendix 5] The information processing apparatus according to any one of Appendices 1 to 4,
   in which the term including the difference is a sum of values obtained by dividing the difference by the target value of the divided portion.
[Appendix 6] The information processing apparatus according to any one of Appendices 1 to 5,
   in which at least one of physical property information of a material constituting the product or design information of the product is also input to the machine learning model.
[Appendix 7] The information processing apparatus according to any one of Appendices 1 to 6,
   in which there are a plurality of types of the prediction values, and
   the machine learning model is prepared for each of the plurality of types of the prediction values.
[Appendix 8] The information processing apparatus according to any one of Appendices 1 to 7,
   in which the product is an endoscope flexible tube including a flexible tube substrate and a resin layer that covers the flexible tube substrate and that is composed of an inner layer and an outer layer of which a thickness ratio is different in an axial direction,
   the divided portion is a portion obtained by dividing the endoscope flexible tube along the axial direction,
   the manufacturing condition includes an extrusion amount per unit time of a resin material of the inner layer and an extrusion amount per unit time of a resin material of the outer layer, as obtained from an extrusion molding machine, and
   the prediction value includes an elasticity value of the divided portion, a thickness of the inner layer of the divided portion, and a thickness of the outer layer of the divided portion.
[Appendix 9] The information processing apparatus according to any one of Appendices 1, 2, and 5 to 7,
   in which the product is a sheet obtained by applying a liquid to a long support to be transported,
   the divided portion is a portion obtained by dividing the sheet along a width direction,
   the manufacturing condition includes a control amount of a tension adjusting roller that is disposed on an upstream side of an application location of the liquid and that adjusts a tension applied to the support at the application location, and
   the prediction value includes a coating thickness of the liquid in the divided portion.
[Appendix 10] The information processing apparatus according to any one of Appendices 1, 2, and 5 to 7,
   in which the product is a cell cultured in a culture tank,
   the divided portion is a portion obtained by dividing the culture tank,
   the manufacturing condition includes a culture condition of the cell, and
   the prediction value includes an index value indicating desirability of a culture environment for the cell.
[Appendix 11] An operation method of an information processing apparatus, the operation method comprising:
   using a machine learning model that outputs a prediction value of quality of a divided portion obtained by dividing an entire product in response to input of a manufacturing condition of the divided portion;
   inputting the manufacturing condition of the divided portion to the machine learning model and outputting the prediction value of the divided portion from the machine learning model; and
   deriving a suitable manufacturing condition under which quality of the entire product reaches a target value by solving an optimization problem seeking a manufacturing condition under which a value of an objective function having a term including a difference between the prediction value of the divided portion and a target value of the quality of the divided portion is minimized.
[Appendix 12] An operation program of an information processing apparatus, the operation program causing a computer to execute:
   using a machine learning model that outputs a prediction value of quality of a divided portion obtained by dividing an entire product in response to input of a manufacturing condition of the divided portion;
   inputting the manufacturing condition of the divided portion to the machine learning model and outputting the prediction value of the divided portion from the machine learning model; and
   deriving a suitable manufacturing condition under which quality of the entire product reaches a target value by solving an optimization problem seeking a manufacturing condition under which a value of an objective function having a term including a difference between the prediction value of the divided portion and a target value of the quality of the divided portion is minimized.

In the technology of the present disclosure, the above-described various embodiments and/or various modification examples may be combined with each other as appropriate. In addition, it is needless to say that the present disclosure is not limited to each of the above-described embodiments, and various configurations can be used without departing from the gist of the present disclosure. Further, the technology of the present disclosure extends to a storage medium that non-transitorily stores a program in addition to the program.

The above descriptions and illustrations are detailed descriptions of portions related to the technology of the present disclosure and are merely examples of the technology of the present disclosure. For example, description related to the above configurations, functions, actions, and effects is description related to an example of configurations, functions, actions, and effects of the parts according to the embodiment of the disclosed technology. Therefore, unnecessary portions may be deleted or new elements may be added or replaced in the above descriptions and illustrations without departing from the gist of the technology of the present disclosure. Further, in order to avoid complications and facilitate understanding of the parts related to the technology of the present disclosure, descriptions of common general knowledge and the like that do not require special descriptions for enabling the implementation of the technology of the present disclosure are omitted, in the contents described and shown above.

In the present specification, the term "A and/or B" is synonymous with the term "at least one of A or B". That is, the term "A and/or B" means only A, only B, or a combination of A and B. In addition, in the present specification, the same approach as "A and/or B" is applied to a case in which three or more matters are represented by connecting the matters with "and/or".

All documents, patent applications, and technical standards mentioned in the present specification are incorporated herein by reference to the same extent as in a case in which each document, each patent application, and each technical standard are specifically and individually described by being incorporated by reference.

## Claims

1. An information processing apparatus comprising:
a processor,
wherein the processor
uses a machine learning model that outputs a prediction value of quality of a divided portion obtained by dividing an entire product in response to input of a manufacturing condition of the divided portion,
inputs the manufacturing condition of the divided portion to the machine learning model and outputs the prediction value of the divided portion from the machine learning model, and
derives a suitable manufacturing condition under which quality of the entire product reaches a target value by solving an optimization problem seeking a manufacturing condition under which a value of an objective function having a term including a difference between the prediction value of the divided portion and a target value of the quality of the divided portion is minimized.

2. The information processing apparatus according to claim 1,
wherein the objective function has a regularization term for accommodating the manufacturing condition of the divided portion in a constraint condition related to a manufacturing condition of the entire product.

3. The information processing apparatus according to claim 2,
wherein the regularization term is a term for smoothing the manufacturing condition of the divided portion.

4. The information processing apparatus according to claim 3,
wherein the regularization term is a sum of second-order differential values of the manufacturing condition of the divided portion.

5. The information processing apparatus according to claim 1,
wherein the term including the difference is a sum of values obtained by dividing the difference by the target value of the divided portion.

6. The information processing apparatus according to claim 1,
wherein at least one of physical property information of a material constituting the product or design information of the product is also input to the machine learning model.

7. The information processing apparatus according to claim 1,
wherein there are a plurality of types of the prediction values, and
the machine learning model is prepared for each of the plurality of types of the prediction values.

8. The information processing apparatus according to claim 1,
wherein the product is an endoscope flexible tube including a flexible tube substrate and a resin layer that covers the flexible tube substrate and that is composed of an inner layer and an outer layer of which a thickness ratio is different in an axial direction,
the divided portion is a portion obtained by dividing the endoscope flexible tube along the axial direction,
the manufacturing condition includes an extrusion amount per unit time of a resin material of the inner layer and an extrusion amount per unit time of a resin material of the outer layer, as obtained from an extrusion molding machine, and
the prediction value includes an elasticity value of the divided portion, a thickness of the inner layer of the divided portion, and a thickness of the outer layer of the divided portion.

9. The information processing apparatus according to claim 1,
wherein the product is a sheet obtained by applying a liquid to a long support to be transported,
the divided portion is a portion obtained by dividing the sheet along a width direction,
the manufacturing condition includes a control amount of a tension adjusting roller that is disposed on an upstream side of an application location of the liquid and that adjusts a tension applied to the support at the application location, and
the prediction value includes a coating thickness of the liquid in the divided portion.

10. The information processing apparatus according to claim 1,
wherein the product is a cell cultured in a culture tank,
the divided portion is a portion obtained by dividing the culture tank,
the manufacturing condition includes a culture condition of the cell, and
the prediction value includes an index value indicating desirability of a culture environment for the cell.

11. An operation method of an information processing apparatus, the operation method comprising:
using a machine learning model that outputs a prediction value of quality of a divided portion obtained by dividing an entire product in response to input of a manufacturing condition of the divided portion;
inputting the manufacturing condition of the divided portion to the machine learning model and outputting the prediction value of the divided portion from the machine learning model; and
deriving a suitable manufacturing condition under which quality of the entire product reaches a target value by solving an optimization problem seeking a manufacturing condition under which a value of an objective function having a term including a difference between the prediction value of the divided portion and a target value of the quality of the divided portion is minimized.

12. An operation program of an information processing apparatus, the operation program causing a computer to execute:
using a machine learning model that outputs a prediction value of quality of a divided portion obtained by dividing an entire product in response to input of a manufacturing condition of the divided portion;
inputting the manufacturing condition of the divided portion to the machine learning model and outputting the prediction value of the divided portion from the machine learning model; and
deriving a suitable manufacturing condition under which quality of the entire product reaches a target value by solving an optimization problem seeking a manufacturing condition under which a value of an objective function having a term including a difference between the prediction value of the divided portion and a target value of the quality of the divided portion is minimized.
